(19) Europäisches Patentamt
European Patent Office
Office européen des brevets

(11) Veröffentlichungsnummer: **0 108 715**
**A1**

# (12) EUROPÄISCHE PATENTANMELDUNG

(21) Anmeldenummer: **83810468.5**

(22) Anmeldetag: **10.10.83**

(51) Int. Cl.³: **C 07 D 223/26**
C 07 D 223/28, C 07 D 223/22
A 61 K 31/55

(30) Priorität: **15.10.82 CH 6020/82**

(43) Veröffentlichungstag der Anmeldung:
**16.05.84 Patentblatt 84/20**

(84) Benannte Vertragsstaaten:
**AT CH DE FR LI LU NL SE**

(71) Anmelder: **CIBA-GEIGY AG**
**Postfach**
**CH-4002 Basel(CH)**

(72) Erfinder: **Storni, Angelo, Dr.**
**Im Feuerbusch 3**
**CH-4310 Rheinfelden(CH)**

(72) Erfinder: **Mondadori, Cesare, Dr.**
**Traugott-Meyer-Strasse 70**
**CH-4147 Aesch(CH)**

## (54) Dibenzazepincarboxamide.

(57) 5H-Debenz[b,f]azepin-5-carboxamide der allgemeinen Formel I

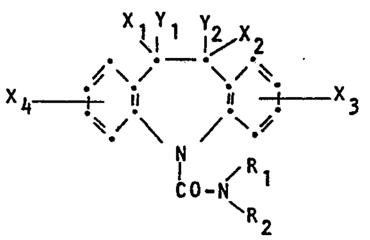

(I),

worin $R_1$ und $R_2$ unabhängig voneinander Wasserstoff oder Niederalkyl, oder unter sich verbunden Niederalkylen oder Aethylenoxyäthylen darstellen, eines der beiden Symbole $X_1$ und $X_2$ und/oder das Symbol $X_3$ Niederalkyl, Niederalkoxy, Niederalkylthio, Niederalkylsulfinyl, Niederalkylsulfonyl oder Trifluormethyl und $X_3$ auch Halogen bis Atomnummer 35, Cyano oder Diniederalkylsulfamoyl bedeutet und das bzw. die beiden restlichen der Symbole $X_1$, $X_2$ und $X_3$ Wasserstoff bedeuten, jedoch zugleich mit Niederalkyl als $X_1$ auch $X_2$ Niederalkyl bedeuten kann, oder, sofern mindestens einer der Reste $R_1$ und $R_2$ von Wasserstoff verschieden ist oder $Y_1$ und $Y_2$ Wasserstoff darstellen, $X_1$, $X_2$ und $X_3$ je Wasserstoff bedeuten können, $X_4$ Wasserstoff bedeutet oder zugleich mit Halogen oder Niederalkyl als $X_3$ ebenfalls einen dieser Reste bedeutet und $Y_1$ und $Y_2$ zusammen eine zusätzliche Bindung oder, sofern $X_1$ und/oder $X_2$ für Niederalkyl oder Wasserstoff stehen, beide Wasserstoff bedeuten, oder, falls jeweils mindestens einer der Reste $R_1$, $R_2$, $X_3$ und $X_4$ von Wasserstoff verschieden ist, $X_1$ auch Halogen bis Atomnummer 35 oder Cyano, $X_2$ Wasserstoff und $Y_1$ und $Y_2$ zusammen eine zusätzliche Bindung, oder $X_1$ und $Y_1$ zusammen den Oxorest und $X_2$ und $Y_2$ je Wasserstoff oder $X_1$ Hydroxy und $X_2$, $Y_1$ und $Y_2$ je Wasserstoff bedeuten können, oder worin $R_1$ Niederalkyl bedeutet, $R_2$, $X_3$, $X_4$, $Y_1$ und $Y_2$ Wasserstoff darstellen und $X_1$ und $X_2$ zusammen für Epoxy oder Epimethylen stehen, weisen nootrope Wirkungen auf und eignen sich zur Behandlung zerebraler Insuffiziens. Die Erfindung betrifft deren Anwendung in einem Behandlungsverfahren zerebraler Insuffiziens, dafür geeignete pharmazeutische Präparate sowie neue Verbindungen der Formel I und Analogieverfahren zu ihrer Herstellung.

EP 0 108 715 A1

CIBA-GEIGY AG
Basel (Schweiz)

4-14150/+

Dibenzazepincarboxamide

Die vorliegende Erfindung betrifft die Verwendung der 5H-Dibenz[b,f]-azepin-5-carboxamide der allgemeinen Formel I

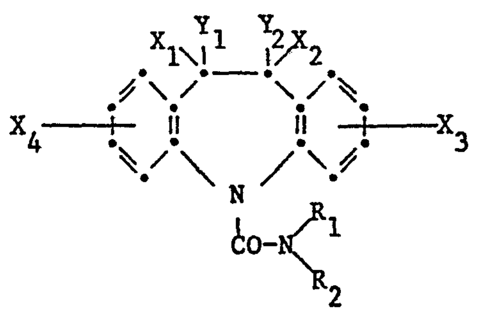

(I),

worin $R_1$ und $R_2$ unabhängig voneinander Wasserstoff oder Niederalkyl, oder unter sich verbunden Niederalkylen oder Aethylenoxyäthylen darstellen, eines der beiden Symbole $X_1$ und $X_2$ und/oder das Symbol $X_3$ Niederalkyl, Niederalkoxy, Niederalkylthio, Niederalkylsulfinyl, Niederalkylsulfonyl oder Trifluormethyl und $X_3$ auch Halogen bis Atomnummer 35, Cyano oder Diniederalkylsulfamoyl bedeutet und das bzw. die beiden restlichen der Symbole $X_1$, $X_2$ und $X_3$ Wasserstoff bedeuten, jedoch zugleich mit Niederalkyl als $X_1$ auch $X_2$ Niederalkyl bedeuten kann, oder, sofern mindestens einer der Reste $R_1$ und $R_2$ von Wasserstoff verschieden ist oder $Y_1$ und $Y_2$ Wasserstoff darstellen, $X_1$, $X_2$ und $X_3$ je Wasserstoff bedeuten können, $X_4$ Wasserstoff bedeutet oder zugleich mit Halogen oder Niederalkyl als $X_3$ ebenfalls einen dieser Reste bedeutet und $Y_1$ und $Y_2$ zusammen eine zusätzliche Bindung oder, sofern $X_1$ und/oder $X_2$ für Niederalkyl oder Wasserstoff stehen, beide Wasserstoff bedeuten, oder, falls jeweils mindestens einer der Reste $R_1$, $R_2$, $X_3$ und $X_4$ von Wasserstoff verschieden ist, $X_1$ bzw. $X_2$ auch Halogen bis Atomnummer 35 oder Cyano, $X_2$ bzw. $X_1$ Wasserstoff und $Y_1$ und $Y_2$ zusammen eine zusätz-

liche Bindung, oder $X_1$ und $Y_1$ zusammen den Oxorest und $X_2$ und $Y_2$ je Wasserstoff oder $X_1$ Hydroxy und $X_2$, $Y_1$ und $Y_2$ je Wasserstoff bedeuten können, oder worin $R_1$ Niederalkyl bedeutet, $R_2$, $X_3$, $X_4$, $Y_1$ und $Y_2$ Wasserstoff darstellen und $X_1$ und $X_2$ zusammen für Epoxy oder Epimethylen stehen. Diese vermögen die amnesiogen Wirkung des Elektroschocks sowohl in einer Dosierung, welche die durch den Elektroschock ausgelösten Konvulsionen völlig oder erheblich, z.B. zu 50% entsprechend der antikonvulsiven $ED_{50}$ als auch teilweise noch in niedrigeren Dosierungen, in mindestens ähnlichem oder sogar stärkerem Ausmass zu vermindern als Piracetam.

Die vorstehend zusammengefassten Befunde können in folgend Einstufen-Lerntest an Mäusen ermittelt werden:

Die Testvorrichtung besteht aus einer grossen Box (35 x 20 x 10 cm), die durch eine Schiebetür mit einer kleinen Box (10 x 10 x 18 cm) verbunden ist. Während die kleine Box durch eine 100 Watt Lampe von oben hell erleuchtet wird, ist die grosse Box dunkel. Der Boden beider Abteile besteht aus einem elektrifizierbaren Gitterrost.

Zum Training werden die Mäuse einzeln in die hell erleuchtete kleine Box gestellt. Da Mäuse eine Spontanpräferenz für Dunkel haben, gehen sie meistens innerhalb von 30 Sekunden ins dunkle Abteil. Sobald sie dieses vollständig betreten haben, wird die Schiebetür geschlossen und ein Fussschock (1 mA, 5 Sekunden) verabreicht. Die Tiere werden darauf sofort aus der Vorrichtung entfernt.

Zur Prüfung des Lerneffektes (Retest) werden die Mäuse nach 24 Stunden wiederum einzeln ins helle Abteil gesetzt und die Latenz, bis sie ganz im Dunkeln sind, gemessen. Ueblicherweise bleiben nun die meisten Tiere über die ganze Beobachtungszeit von 150 Sekunden im hellen Abteil.

Der Lerneffekt wird weitgehend aufgehoben bzw. die Erinnerung an den Fussschock mindestens partiell ausgelöscht, wenn als amnesiogene Behandlung unmittelbar anschliessend an den Fussschock des Trainingsdurchgangs eine temporale Elektroschockbehandlung angeschlossen wird. Parameter des Elektroschocks: 50 mA, 0,4 Sekunden, 50 Hz, 0,6 msec Pulsbreite.

Zur Prüfung und zum Vergleich ihrer Schutzwirkung gegenüber der amnesiogenen Wirkung des Elektroschocks werden die Testsubstanzen jeweils 30 Minuten vor dem Training als Suspension in Methocel intraperitoneal verabreicht und die Tiere unmittelbar nach dem Training der Elektroschockbehandlung unterzogen. 24 Stunden später wird anhand der Verweildauer in der beleuchteten Box das Ausmass des noch erhalten gebliebenen Lerneffektes gemessen und mit demjenigen der anderen Testsubstanzen wie auch von Kontrolltieren mit blosser Methocel-Verabreichung und Training ohne, wie auch solchen mit anschliessender Elektroschockbehandlung, verglichen. Als gemäss dem Mann-Whitney U-Test noch signifikant wirksame Dosen, d.h. $DE_{min}$ wurden z.B. für 10-Methylthio-5H-dibenz[b,f]azepin-5-carboxamid 0,1 mg/kg i.p. und für 3-Methylsulfonyl-5H-dibenz[b,f]azepin-5-carboxamid ebenfalls 0,1 mg/kg. i.p. ermittelt.

Als Literatur über ähnliche Versuche seien genannt: S.J. Sara und D.Lefevre, Psychopharmacologia 25, 32-40 (1972), Hypoxia-induced amnesia in one-trial learning and pharmacological protection by piracetam; sowie Boggan, W.O., und Schlesinger, K., in Behavioral Biology 12, 127-134 (1974).

Von den Verbindungen der oben definierten allgemeinen Formel I sind solche, in denen $R_1$ und $R_2$ unabhängig voneinander Wasserstoff oder Niederalkyl oder unter sich verbunden Niederalkylen oder Aethylenoxyäthylen darstellen, eines der Symbole $X_1$ und $X_2$ Niederalkylthio,

Niederalkylsulfinyl, Niederalkylsulfonyl, Trifluormethyl, Niederalkoxy, Halogen der Atomnummer bis und mit 35 oder Cyano und das andere Wasserstoff, Niederalkyl, Niederalkoxy, Niederalkylthio, Niederalkylsulfinyl, Niederalkylsulfonyl oder Trifluormethyl bedeutet oder beide Symbole $X_1$ und $X_2$ für Wasserstoff stehen, $X_3$ Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl darstellt oder, sofern mindestens einer der Reste $X_1$ und $X_2$ von Wasserstoff verschieden ist und zugleich mit Halogen oder Cyano als $X_1$ bzw. $X_2$ und Wasserstoff als $X_2$ bzw. $X_1$ $R_1$ Wasserstoff oder Niederalkyl und $R_2$ Niederalkyl oder $R_1$ gemeinsam mit $R_2$ Niederalkylen oder Aethylenoxyäthylen und zugleich mit Niederalkoxy als $X_1$ bzw. $X_2$ und Wasserstoff als $X_2$ bzw. $X_1$ $R_1$ gemeinsam mit $R_2$ Niederalkylen oder Aethylenoxyäthylen bedeutet, auch Wasserstoff bedeuten kann, $X_4$ Wasserstoff bedeutet oder zugleich mit Niederalkyl oder Halogen als $X_3$ ebenfalls einen dieser Reste darstellen kann, und die Symbole $Y_1$ und $Y_2$, sofern eines der Symbole $X_1$ und $X_2$ Niederalkylthio, Niederalkylsulfinyl, Niederalkylsulfonyl oder Trifluormethyl und das andere Wasserstoff bedeutet oder beide Wasserstoff darstellen, jeweils für Wasserstoff stehen oder, sofern mindestens einer der Reste $R_1$, $R_2$, $X_1$ und $X_2$ von Wasserstoff oder $X_3$ von Cyano oder mindestens einer der Reste $X_3$ und $X_4$ von Halogen verschieden ist, gemeinsam eine zusätzliche Bindung darstellen, oder worin $R_1$ und $R_2$ gemeinsam für Niederalkylen oder Aethylenoxyäthylen stehen und $X_1$ gemeinsam mit $Y_1$ Oxo und $X_2$ sowie $Y_2$ Wasserstoff bzw. $X_1$ Hydroxy und $X_2$, $Y_1$ sowie $Y_2$ Wasserstoff darstellt oder $R_1$ und $R_2$ unabhängig voneinander Niederalkyl oder gemeinsam Niederalkylen oder Aethylenoxyäthylen bedeuten, $X_1$ gemeinsam mit $X_2$ Epoxy oder Epimethylen und $Y_1$ sowie $Y_2$ jeweils Wasserstoff darstellt, $X_3$ für Wasserstoff, Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl steht, und $X_4$ Wasserstoff bedeutet oder zugleich mit Niederalkyl oder Halogen als $X_3$ ebenfalls einen dieser Reste darstellt, neu.

Verbindungen der allgemeinen Formel I, in denen $X_1$ Niederalkoxy bedeutet, $R_1$ und $R_2$ Wasserstoff oder Niederalkyl, $X_2$, $X_3$ und $X_4$ je Wasserstoff und $Y_1$ und $Y_2$ zusammen eine zusätzliche Bindung bedeuten, sind in der CH-Patentschrift Nr. 500.196 als Zwischenprodukte beschrieben, namentlich 10-Methoxy-5H-dibenz[b,f]azepin-5-carboxamid, N-Methyl-, N,N-Dimethyl-, N-Butyl- und N,N-Dibutyl-10-methoxy-5H-dibenz[b,f]-azepin-5-carboxamid.

Ferner sind in den CH-Patentschriften Nr. 325.662, 403.767, 500.101 und 501.196, der GB-Patentschrift Nr. 906.452, der EP-Offenlegungsschrift Nr. 50.589 sowie in der BE-Patentschrift Nr. 892.882 Verbindungen der Formel I, worin $R_1$ und $R_2$ die angegebenen Bedeutungen haben, $X_1$ und $X_2$ Wasserstoff oder Niederalkyl bzw., sofern $R_1$ und $R_2$ für Wasserstoff oder Niederalkyl stehen, $X_1$ Hydroxy und $X_2$ Wasserstoff bedeuten, $X_3$ und $X_4$ Wasserstoff oder, sofern $X_1$ und/oder $X_2$ Wasserstoff oder Niederalkyl bedeuten, $X_3$ Halogen, Niederalkyl oder Niederalkoxy und $X_4$ Halogen oder Niederalkyl ist, wobei $Y_1$ und $Y_2$ jeweils Wasserstoff oder gemeinsam eine zusätzliche Bindung darstellen, als Antikonvulsiva beschrieben. Ferner sind in der US-Patentschrift Nr. 3.842.091 und in der JP-Patentanmeldung Nr. 7308631 Verbindungen der Formel I, worin $R_1$ Niederalkyl, $X_1$ gemeinsam mit $X_2$ Epoxy oder Epimethylen und $R_2$, $X_3$, $X_4$, $Y_1$ und $Y_2$ Wasserstoff bedeuten, als Antikonvulsiva beschrieben. Weiterhin sind in der US-Patentschrift Nr. 4.235.895, der BE-Patentschrift Nr. 833.852 sowie der EP-Patentanmeldung Nr. 50.589 Verbindungen der Formel I, worin $R_1$, $R_2$, $X_3$ und $X_4$ Wasserstoff bedeutet, $X_1$ Cyano oder Hydroxy und $X_2$ Wasserstoff bzw. $X_1$ Halogen und $X_2$ Wasserstoff oder Halogen bzw. $X_1$ und $X_2$ Wasserstoff und $X_3$ Cyano ist und $Y_1$ und $Y_2$ gemeinsam eine zusätzliche Bindung oder, sofern $X_1$ Hydroxy ist, Wasserstoff darstellen, als Antikonvulsiva beschrieben und einige davon in der nicht vorpublizierten BE-Patentschrift Nr. 892.882 als Nootropika vorgeschlagen worden.

Einige dieser Verbindungen sind in diesen Patentschriften auch spezifisch beschrieben, z.B. 10,11-Dihydro-5H-dibenz[b,f]azepin-5-carboxamid (CH-PS 325,662), 3,7-Dichlor-5H-dibenz-[b,f]azepin-5-carboxamid, 3,7-Dibrom-5H-dibenz[b,f]azepin-5-carboxamid, N,N-Diäthyl-5H-dibenz[b,f]-azepin-5-carboxamid, 5-[(1-Piperdinyl)-carbonyl]-, 5-[(1-Pyrrolidinyl)-carbonyl]- und 5-[(4-Morpholinyl)-carbonyl]-5H-dibenz[b,f]azepin, (alle GB-PS 906,452) 10,11-Dihydro-10-methyl-5H-dibenz[b,f]azepin-5-carboxamid, 10-Methyl-5H-dibenz[b,f]azepin-5-carboxamid und 10,11-Dimethyl-5H-dibenz[b,f]azepin-5-carboxamid (alle CH-PS 403,767), N-Methyl-, N,N-Dimethyl-, N-Butyl- und N,N-Dibutyl-10,11-dihydro-10-oxo-5H-dibenz[b,f]-azepin-5-carboxamid (alle als Endstoffe in der CH-PS 500,196), N-Methyl-, N,N-Dimethyl- und N-Butyl-10,11-dihydro-10-hydroxy-5H-dibenz[b,f]azepin-5-carboxamid (alle CH-PS 505,101), N-Methyl-1,1a,6,10b-tetrahydro-cyclopropa[d]dibenz[b,f]azepin-6-carboxamid (JP-OLS 7308631) und 1a,10b-Dihydro-N-propyl-6H-dibenz[b,f]oxiren[d]azepin-6-carboxamid (DE-OLS 2246842). Auch diese Verbindungen kommen als Wirkstoffe für pharmazeutische Präparate und zur Anwendung im erfindungsgemässen Verfahren in Betracht. Besonders wichtig sind als erfindungsgemässe Wirkstoffe die in den Herstellungs- und Präparatebeispielen genannten Verbindungen, insbesondere die beiden weiter oben angegebenen.

Die Verbindungen der Formel I können in Verfahren zur Prophylaxe oder Therapie von zerebraler Insuffizienz, insbesondere von Gedächtnisstörungen verschiedener Genese, wie senile Demenz, Multi-infarkt-Demenz oder Demenz vom Alzheimer-Typ, ferner von Folgezuständen von Hirntraumen oder Apoplexie, Verwendung finden.

Die Dosierung richtet sich dabei nach der Art der Störung, dem individuellen Zustand, Alter und Gewicht des Patienten und der Dauer der Behandlung und liegt insbesondere zwischen etwa 0,2 und etwa 17 mg/kg pro Tag bzw. etwa 15 bis 1200 mg pro Tag für erwachsene Personen von normalem Gewicht von etwa 70 kg.

Ebenfalls Gegenstand der Erfindung sind pharmazeutische Präparate, z.B. solcher zur Prophylaxe oder Therapie von zerebraler Insuffizienz, welche mindestens eine Verbindung der oben definierten allgemeinen Formel I, gegebenenfalls zusammen mit pharmazeutischen Trägerstoffen enthalten.

Im erfindungsgemässen Behandlungsverfahren werden beispielsweise Verbindungen der Formel I, worin $R_1$ und $R_2$ unabhängig voneinander Wasserstoff oder Niederalkyl, oder unter sich verbunden Niederalkylen oder Aethylenoxyäthylen darstellen, die Symbole $X_1$ und $X_2$ unabhängig voneinander Wasserstoff oder Niederalkyl bedeuten, das Symbol $X_3$, sofern $X_1$ und $X_2$ Wasserstoff darstellen, für Halogen oder Atomnummer bis und mit 35 oder zugleich mit Wasserstoff als $R_1$ und $R_2$ für Cyano oder, sofern $X_1$ und/oder $X_2$ Niederalkyl darstellt, für Wasserstoff, Niederalkyl, Niederalkoxy oder Halogen der Atomnummer bis und mit 35, ferner für Niederalkylthio, Niederalkylsulfinyl, Niederalkylsulfonyl, Trifluormethyl, Cyano oder Diniederalkylsulfamoyl steht, $X_4$ Wasserstoff oder zugleich mit Halogen als $X_3$ ebenfalls Halogen bzw. zugleich mit Niederalkyl als $X_3$ ebenfalls Halogen oder Niederalkyl bedeutet und $Y_1$ und $Y_2$ gemeinsam für eine zusätzliche Bindung oder, sofern $X_1$ und $X_2$ Wasserstoff darstellt, ebenfalls für Wasserstoff stehen, oder worin $R_1$ und $R_2$ Niederalkyl bedeuten und $X_1$ gemeinsam mit $Y_1$ Oxo und $X_2$ sowie $Y_2$ Wasserstoff bzw. $X_1$ Hydroxy und $Y_1$, $X_2$ sowie $Y_2$ Wasserstoff bedeuten und $X_3$ und $X_4$ Wasserstoff darstellen, oder worin $R_1$ Wasserstoff und $R_2$ Niederalkyl bedeutet, $X_1$ gemeinsam mit $X_2$ Epoxy oder Epimethylen darstellt und $Y_1$, $Y_2$, $X_3$ und $X_4$ für Wasserstoff stehen.

In erster Linie werden solche Verbindungen der Formel I verwendet, worin $R_1$ und $R_2$ unabhängig voneinander Wasserstoff oder Niederalkyl, oder unter sich verbunden Niederalkylen oder Aethylenoxyäthylen darstellen, eines der Symbole $X_1$ und $X_2$ Wasserstoff oder Niederalkyl und

das andere Niederalkyl oder, sofern mindestens einer der Reste $R_1$ und $R_2$ von Wasserstoff verschieden ist, Wasserstoff bedeutet oder $X_1$ und $X_2$ gemeinsam Epoxy oder Epimethylen darstellen, $X_3$ für Wasserstoff oder für Halogen der Atomnummer bis und mit 35, Niederalkyl, Niederalkoxy, ferner Niederalkylthio, Niederalkylsulfinyl, Niederalkylsulfonyl, Trifluormethyl, Cyano oder Diniederalkylsulfamoyl steht, $X_4$ Wasserstoff bedeutet oder zugleich mit Halogen oder Niederalkyl als $X_3$ ebenfalls einen dieser Reste bedeuten kann und $Y_1$ und $Y_2$ jeweils Wasserstoff oder, sofern $X_1$ und $X_2$ Wasserstoff und/oder Niederalkyl darstellen, zusammen eine zusätzliche Bindung bedeuten, namentlich 10,11-Dihydro-10-methyl- und 10,11-Dihydro-10,11-dimethyl-5H-dibenz[b,f]azepin-5-carboxamid, 10-Methyl-5H-dibenz[b,f]azepin-5-carboxamid und 10,11-Dimethyl-5H-dibenz[b,f]azepin-5-carboxamid, 10,11-Dihydro-5H-dibenz[b,f]azepin-5-carboxamid, N,N-Diäthyl-5H-dibenz[b,f]azepin-5-carboxamid und 5-[(1-Piperidinyl)-carbonyl]-, 5-[(1-Pyrrolidinyl)-carbonyl]- und 5-[(4-Morpholinyl)-carbonyl]-5H-dibenz[b,f]azepin, N-Methyl-1,1a,10b-tetrahydrocyclopropa[d]dibenz-[b,f]azepin-6-carboxamid und N-Propyl-1a,10b-dihydro-6H-dibenz[b,f]-oxiren[d]azepin-6-carboxamid.

Vorzugsweise werden Verbindungen der Formel I verwendet, worin $R_1$ und $R_2$ für Wasserstoff stehen, eines der Symbole $X_1$ und $X_2$ Halogen der Atomnummer bis und mit 35 oder Cyano und das andere Wasserstoff bedeutet, $X_3$ für Halogen der Atomnummer bis und mit 35, Niederalkyl, Niederalkoxy, ferner Niederalkylthio, Niederalkylsulfinyl, Niederalkylsulfonyl, Trifluormethyl, Cyano oder Diniederalkylsulfamoyl steht, $X_4$ Wasserstoff bedeutet oder zugleich mit Halogen oder Niederalkyl als $X_3$ ebenfalls einen dieser Reste bedeuten kann, und $Y_1$ und $Y_2$ zusammen eine zusätzliche Bindung darstellen, namentlich 3,7-Dichlor- und 3,7-Dibrom-5H-dibenz[b,f]azepin-5-carboxamid, verwendet.

Vorzugsweise werden ebenso Verbindungen der Formel I verwendet, worin $R_1$ und $R_2$ unabhängig voneinander Wasserstoff oder Niederalkyl oder unter sich verbunden Niederalkylen oder Aethylenoxyäthylen darstellen, $X_1$ und $Y_1$ gemeinsam Oxo und $X_2$ und $Y_2$ jeweils Wasserstoff bzw. $X_1$ Hydroxy und $X_2$, $Y_1$ und $Y_2$ jeweils Wasserstoff darstellen, $X_3$ für Halogen der Atomnummer bis und mit 35, Niederalkyl, Niederalkoxy, ferner Niederalkylthio, Niederalkylsulfinyl, Niederalkylsulfonyl, Trifluormethyl, Cyano oder Diniederalkylsulfamoyl oder, sofern mindestens einer der Reste $R_1$ und $R_2$ von Wasserstoff verschieden ist, für Wasserstoff steht und $X_4$ Wasserstoff bedeutet oder zugleich mit Halogen oder Niederalkyl als $X_3$ ebenfalls einen dieser Reste bedeuten kann, namentlich N-Methyl-, N,N-Dimethyl-, N-Butyl- und N,N-Dibutyl-10,11-dihydro-10-oxo-5H-dibenz[b,f]azepin-5-carboxamid sowie N-Methyl-, N,N-Dimethyl- und N-Butyl-10,11-dihydro-10-hydroxy-5H-dibenz[b,f]azepin-5-carboxamid verwendet.

Vorzugsweise werden weiterhin Verbindungen der Formel I verwendet, worin $R_1$ und $R_2$ unabhängig voneinander Wasserstoff oder Niederalkyl oder unter sich verbunden Niederalkylen oder Aethylenoxyäthylen darstellen, $X_1$ und $X_2$ für Wasserstoff stehen und $X_3$ sowie $X_4$ für Halogen der Atomnummer bis und mit 35 stehen oder $R_1$, $R_2$, $X_1$, $X_2$ und $X_4$ Wasserstoff bedeuten und $X_3$ Cyano ist, wobei jeweils $Y_1$ und $Y_2$ gemeinsam eine zusätzliche Bindung darstellen, namentlich 2-Cyano- und 3-Cyano-5H-dibenz[b,f]azepin-5-carboxamid, sowie 3,7-Dichlor- und 3,7-Dibrom-5H-dibenz[b,f]azepin-5-carboxamid.

Die Erfindung betrifft ferner die vorstehend als neu bzw. als bekannte Zwischenprodukte bezeichneten Verbindungen der Formel I, beispielsweise solche, in denen $R_1$ und $R_2$ Wasserstoff oder Niederalkyl bedeuten, $X_1$ Niederalkoxy ist, $X_2$, $X_3$ und $X_4$ Wasserstoff darstellen und $Y_1$ und $Y_2$ gemeinsam eine zusätzliche Bindung darstellen zur Anwendung in einem Verfahren zur therapeutischen Behandlung des menschlichen oder tierischen

Körpers, beispielsweise in dem eingangs genannten Behandlungsverfahren, ihre Verwendung als Arzneimittelwirkstoffe und diese enthaltende pharmazeutische Präparate, vorzugsweise nootrope pharmazeutische Präparate. Im Vordergrund stehen dabei neben den in den Beispielen spezifisch genannten Verbindungen der Formel I, insbesondere 10-Methoxy-5H dibenz[[b,f]azepin-5-carboxamid, N-Methyl-, N,N-Dimethyl-, N-Butyl- und N,N-Dibutyl-10-methoxy-5H-dibenz[b,f]azepin-5-carboxamid.

Die Erfindung betrifft weiterhin die vorstehend als neu bezeichneten Verbindungen der Formel I selbst, Verfahren zu ihrer Herstellung, sie enthaltende pharmazeutische Präparate und ihre Verwendung als Arzneimittelwirkstoffe.

Die Erfindung betrifft beispielsweise Verbindungen der Formel I, in denen $R_1$ und $R_2$ unabhängig voneinander Wasserstoff oder Niederalkyl oder unter sich verbunden Niederalkylen oder Aethylenoxyäthylen, eines der beiden Symbole $X_1$ und $X_2$ und/oder das Symbol $X_3$ Niederalkylthio, Niederalkylsulfinyl, Niederalkylsulfonyl oder Trifluormethyl und $X_3$ auch Halogen bis Atomnummer 35, Diniederalkylsulfamoyl, Niederalkyl, Cyano oder Niederalkoxy bedeutet und das bzw. die beiden restlichen der Symbole $X_1$, $X_2$ und $X_3$ Wasserstoff bedeuten, jedoch $X_1$ oder $X_2$, sofern mindestens einer der Reste $R_1$ und $R_2$ von Wasserstoff und Niederalkyl oder einer der Reste $X_3$ und $X_4$ von Wasserstoff verschieden ist und $Y_1$ und $Y_2$ eine zusätzliche Bindung darstellen, auch Niederalkoxy und $X_2$ bzw. $X_1$ Wasserstoff bedeuten kann, $X_4$ Wasserstoff bedeutet oder zugleich mit Halogen oder Niederalkyl als $X_3$ ebenfalls einen dieser Reste bedeuten kann, $Y_1$ und $Y_2$ zusammen eine zusätzliche Bindung bedeuten oder zugleich mit Wasserstoff als $X_1$ und $X_2$ auch je Wasserstoff bedeuten können, oder falls mindestens einer der Reste $R_1$ und $R_2$ von Wasserstoff verschieden ist und $X_3$ und $X_4$ Wasserstoff bedeuten, $X_1$ auch Halogen bis Atomnummer 35 oder Cyano, $X_2$ Wasserstoff und $Y_1$ und $Y_2$ zusammen eine zusätzliche Bindung bedeuten können.

Die Erfindung betrifft in erster Linie solche Verbindungen der Formel I, worin $R_1$ und $R_2$ unabhängig voneinander Wasserstoff oder Niederalkyl oder unter sich verbunden Niederalkylen oder Aethylenoxyäthylen bedeuten, eines der beiden Symbole $X_1$ und $X_2$ Niederalkylthio, Niederalkylsulfinyl, Niederalkylsulfonyl oder Trifluormethyl und das andere Wasserstoff bedeutet, $X_3$ Wasserstoff, Niederalkylthio, Niederalkylsulfinyl, Niederalkylsulfonyl, Trifluormethyl, Cyano, Halogen bis Atomnummer 35, Diniederalkylsulfamoyl, Niederalkyl oder Niederalkoxy bedeutet, $X_4$ Wasserstoff bedeutet oder zugleich mit Halogen oder Niederalkyl als $X_3$ ebenfalls einen dieser Reste bedeuten kann und $Y_1$ und $Y_2$ zusammen eine zusätzliche Bindung oder jeweils Wasserstoff darstellen.

Die Erfindung betrifft in erster Linie ebenso Verbindungen der Formel I, worin $R_1$ und $R_2$ unabhängig voneinander Wasserstoff oder Niederalkyl oder unter sich verbunden Niederalkylen oder Aethylenoxyäthylen, $X_1$ und $X_2$ Wasserstoff bedeuten, $X_3$ Halogen der Atomnummer bis und mit 35, Niederalkyl, Niederalkoxy, Niederalkylthio, Niederalkylsulfinyl, Niederalkylsulfonyl, Trifluormethyl, Cyano oder Diniederalkylsulfamoyl bedeutet, $X_4$ Wasserstoff bedeutet oder zugleich mit Halogen oder Niederalkyl als $X_3$ ebenfalls einen dieser Reste bedeuten kann und $Y_1$ und $Y_2$ jeweils Wasserstoff oder, sofern mindestens einer der Reste $R_1$ und $R_2$ von Wasserstoff oder $X_3$ von Cyano oder $X_4$ von Halogen verschieden ist, zusammen eine zusätzliche Bindung darstellen.

Die Erfindung betrifft in erster Linie weiterhin Verbindungen der Formel I, worin eines der Symbole $R_1$ und $R_2$ Niederalkyl und das andere Wasserstoff bedeutet oder beide für Niederalkyl oder gemeinsam für Niederalkylen oder Aethylenoxyäthylen stehen, eines der Symbole $X_1$ und $X_2$ Halogen der Atomnummer bis und mit 35 oder Cyano und das andere ebenso wie $X_3$ und $X_4$ für Wasserstoff stehen, wobei $Y_1$ und $Y_2$ gemeinsam eine zusätzliche Bindung darstellen.

Die Erfindung betrifft in erster Linie ebenfalls Verbindungen der Formel I, worin $R_1$ und $R_2$ unabhängig voneinander Wasserstoff oder Niederalkyl oder unter sich verbunden Niederalkylen oder Aethylenoxyäthylen darstellen, $X_1$ und $Y_1$ gemeinsam Oxo und $X_2$ und $Y_2$ jeweils Wasserstoff bzw. $X_1$ Niederalkoxy oder Hydroxy und $X_2$, $Y_1$ und $Y_2$ jeweils Wasserstoff darstellen, $X_3$ für Halogen der Atomnummer bis und mit 35, Niederalkyl, Niederalkoxy, ferner Niederalkylthio, Niederalkylsulfinyl, Niederalkylsulfonyl, Trifluormethyl, Cyano oder Diniederalkylsulfamoyl oder, sofern $R_1$ und $R_2$ gemeinsam Niederalkylen oder Aethylenoxyäthylen darstellen, für Wasserstoff steht und $X_4$ Wasserstoff bedeutet oder zugleich mit Halogen oder Niederalkyl als $X_3$ ebenfalls einen dieser Reste bedeuten kann.

Die Erfindung betrifft vor allem Verbindungen der Formel I, worin einerseits $R_1$, $R_2$, $X_1$ und $X_2$ Wasserstoff bedeuten, $X_3$ Niederalkylthio, Niederalkylsulfinyl oder Niederalkylsulfonyl mit jeweils bis und mit 4 C-Atomen, wie Methylthio oder Methylsulfonyl, Diniederalkylsulfamoyl mit bis und mit 4 C-Atomen in jedem Alkylteil, wie Dimethylsulfamoyl, Niederalkyl oder Niederalkoxy mit bis und mit 4 C-Atomen, wie Methyl oder Methoxy, Halogen der Atomnummer bis und mit 35, wie Chlor, oder Trifluormethyl bedeutet, $X_4$ für Wasserstoff steht oder zugleich mit Niederalkyl oder Halogen als $X_3$ auch für Niederalkyl stehen kann und $Y_1$ und $Y_2$ jeweils Wasserstoff oder zusammen eine zusätzliche Bindung darstellen, oder worin andererseits $R_1$ und $R_2$ unabhängig voneinander Wasserstoff oder Niederalkyl darstellen, welches je bis und mit 4 C-Atome aufweist und z.B. Methyl bedeutet, einer der Reste $X_1$ und $X_2$ Niederalkylthio, Niederalkylsulfinyl oder Niederalkylsulfonyl mit jeweils bis und mit 4 C-Atomen, wie Methylthio oder Methylsulfonyl, Trifluormethyl oder, sofern mindestens einer der Reste $R_1$ und $R_2$ Niederalkyl darstellt, Halogen der Atomnummer bis und mit 35, wie Chlor oder Brom oder Cyano bedeutet und der andere sowie $X_3$ und $X_4$ Wasserstoff ist und $Y_1$ und $Y_2$ zusammen eine zusätzliche Bindung darstellen.

Die Erfindung betrifft insbesondere Verbindungen der Formel I, worin 5 der Symbole $R_1$, $R_2$, $X_1$, $X_2$, $X_3$ und $X_4$ Wasserstoff darstellen und ein davon verschiedener Rest $R_1$ und/oder $R_2$ Niederalkyl mit bis und mit 4 C-Atomen, wie Methyl oder Butyl, ein davon verschiedener Rest $X_1$ oder $X_2$ Niederalkylthio, Niederalkylsulfinyl oder Niederalkylsulfonyl mit jeweils bis und mit 4 C-Atomen, wie Methylthio oder Methylsulfonyl, oder Trifluormethyl bzw. ein davon verschiedener Rest $X_3$ Niederalkylthio, Niederalkylsulfinyl oder Niederalkylsulfonyl mit jeweils bis und mit 4 C-Atomen, wie Methylthio oder Methylsulfonyl, Diniederalkylsulfamoyl mit bis und mit 4 C-Atomen in jedem Alkylteil, wie Dimethylsulfamoyl, Niederalkyl oder Niederalkoxy mit bis und mit 4 C-Atomen, wie Methyl oder Methoxy, Halogen der Atomnummer bis und mit 35, wie Chlor, oder Trifluormethyl bedeutet und $Y_1$ und $Y_2$ zusammen eine zusätzliche Bindung darstellen.

Die Erfindung betrifft, wie bereits erwähnt, namentlich die in den Herstellungsbeispielen beschriebenen neuen Verbindungen.

Vor- und nachstehend werden unter niederen Resten oder Verbindungen vorzugsweise solche mit höchstens 7, insbesondere höchstens 4, Kohlenstoffatomen verstanden. In den Verbindungen der allgemeinen Formel I können die verwendeten Allgemeinbegriffe beispielsweise durch die nachstehend genannten Reste verkörpert sein: Niederalkyl ist z.B. Propyl, Butyl, Isobutyl, Pentyl, Isopentyl, Neopentyl, Hexyl, Heptyl und vor allem Methyl oder insbesondere als $X_3$ neben Wasserstoff als $X_4$ Aethyl, Niederalkylen ist z.B. 4- bis 6-gliedrig und bildet dementsprechend zusammen mit dem anliegenden Stickstoffatom, z.B. 1-Pyrrolidinyl, 2,5- und 3,4-Dimethyl-1-pyrrolidinyl, 1-Piperidinyl, 4-Methyl-, 2,6-Dimethyl- oder 4,4-Dimethyl-1-piperidinyl oder Hexahydro-1H-azepin-1-yl. Niederalkoxy ist z.B. Aethoxy, Propoxy, Isopropoxy, Butoxy, Isobutoxy, Pentyloxy, Isopentyloxy, Neopentyloxy, Hexyloxy, Heptyloxy und vor allem Methoxy. Niederalkylthio ist z.B. Aethylthio, Propylthio, Isopropylthio, Butylthio oder Isobutylthio

und vor allem Methylthio, Niederalkylsulfinyl z.B. Methylsulfinyl oder Aethylsulfinyl und Niederalkylsulfonyl z.B. Aethylsulfonyl, Propylsulfonyl, Isopropylsulfonyl, Butylsulfonyl oder Isobutylsulfonyl und vor allem Methylsulfonyl.Halogen bis Atomnummer 35 ist Fluor, Brom und vor allem Chlor, und Diniederalkylsulfamoyl z.B. Dimethylsulfamoyl oder Diäthylsulfamoyl. Ein Substituent $X_3$ befindet sich beispielsweise in 2-Stellung oder insbesondere in 3-Stellung. Falls $X_4$ gleich wie $X_3$ Niederalkyl oder Halogen bedeutet, befindet sich $X_4$ vorzugsweise in gleichem Abstand vom Stickstoffatom des mittleren Ringes, d.h. z.B. befinden sich Methylgruppen oder Chloratome $X_3$ und $X_4$ in 2,8- oder vorzugsweise in 3,7-Stellung des Tricyclus.

Erfindungsgemässe pharmazeutische Präparate können einzeldosierte Arzneiformen, d.h. Doseneinheitsformen zur oralen Verabreichung, wie Tabletten, Dragées oder Kapseln, oder zur rektalen Verabreichung wie Suppositorien, oder nicht-einzeldosierte Arzneiformen zur oralen Verabreichung, wie Sirups, sein. Doseneinheitsformen enthalten eine Verbindung der allgemeinen Formel I in einer Menge, die für die Einnahme der obengenannten Tagesdosis bei einer ein- oder mehrmaligen, vorzugsweise höchstens dreimaligen, insbesondere zweimaligen Verabreichung von jeweils vorzugsweise einer oder zwei Doseneinheiten geeignet ist, d.h. die gesamte bzw. ein Drittel oder insbesondere die Hälfte einer Tagesdosis, bzw. die Hälfte, ein Sechstel oder ein Viertel, entsprechend etwa 2,5 bis etwa 600 mg, vorzugsweise etwa 5 bis etwa 300 mg. Anstelle einer einzigen Verbindung der allgemeinen Formel I können auch mehrere gemeinsam verabreicht werden, wobei die Gesamtdosen und -mengen ebenfalls im oben für Einzelverbindungen angegebenen Bereich bleiben.

Teilbare Doseneinheitsformen, wie brechbare Tabletten, und solche mit verzögerter Wirkstoffabgabe können auch höhere Wirkstoffmengen enthalten und anderseits Doseneinheitsformen zur Verabreichung an Kinder auch entsprechend niedrigere Wirkstoffmengen.

Nichteinzeldosierte orale Arzneiformen, wie Sirups, enthalten denjenigen von einzelnen Doseneinheiten entsprechende Mengen der Wirkstoffe in passenden Mengen bzw. Volumeneinheiten von z.B. 2,5, 5 oder 10 ml.

Erfindungsgemäss anwendbare pharmazeutische Präparate enthalten eine oder gegebenenfalls mehrere Verbindungen der allgemeinen Formel I vorzugsweise zusammen mit anorganischen oder organischen, festen oder flüssigen, pharmazeutisch anwendbaren Trägerstoffen, die sich zur enteralen, z.B. oralen Verabreichung eignen. Zur Herstellung von Tabletten oder Dragée-Kernen kombiniert man die Wirkstoffe z.B. mit festen pulverförmigen Trägerstoffen, wie Lactose, Dextrose, Saccharose, Mannit, Sorbit, Cellulose und/oder Glycin, gegebenenfalls unter Zusatz von Gleit- und Schmiermitteln, z.B. Siliciumdioxid, Talk, Stearinsäure oder Salze davon, wie Magnesium- oder Calciumstearat, und/oder Polyäthylenglykol, gegebenenfalls unter Zusatz von Bindemitteln, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. wiederum Stärken, Agar, Alginsäure oder Salze davon, wie Natriumalginat, und/oder Brausemischungen, oder Adsorptionsmittel, Farbstoffe, Geschmackstoffe und Süssmittel. Die Dragée-Kerne überzieht man beispielsweise mit konzentrierten Zuckerlösungen, welche z.B. noch arabischen Gummi, Talk und/oder Titandioxid enthalten können, oder mit einem Lack, der in leicht-flüchtigen organischen Lösungsmitteln oder Lösungsmittelgemischen gelöst ist. Diesen Ueberzügen können Farbstoffe zugefügt werden, z.B. zur Kennzeichnung verschiedener Wirkstoffdosen.

Als weitere orale Doseneinheitsformen eignen sich Steckkapseln aus Gelatine sowie weiche, geschlossene Kapseln aus Gelatine und einem Weichmacher, wie Glycerin. Die Steckkapseln enthalten den Wirkstoff vorzugsweise als Granulat, z.B. in Mischung mit Füllstoffen, wie

Maisstärke, und/oder Gleitmitteln, wie Talk oder Magnesiumstearat, und gegebenenfalls Stabilisatoren, wie z.B. Ascorbinsäure. In weichen Kapseln ist der Wirkstoff vorzugsweise in geeigneten Flüssigkeiten, wie flüssigen Polyäthylenglykolen, gelöst oder suspendiert, wobei ebenfalls Stabilisatoren zugefügt sein können.

Als nicht einzel-dosierte Arzneiformen kommen insbesondere in üblicher Weise hergestellte Sirups, welche einen Wirkstoff der allgemeinen Formel I vorzugsweise in suspendierter Form enthalten, in Betracht.

Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier- oder Lösungsverfahren, hergestellt und enthalten von etwa 5% bis 100%, insbesondere von etwa 10% bis etwa 80%, einer oder gegebenenfalls mehrerer Verbindungen der allgemeinen Formel I.

Die vorliegende Erfindung betrifft auch die weiter oben generell als neu bezeichneten Verbindungen der allgemeinen Formel I, sowie die weiteren, in den oben angeführten Patentschriften nicht spezifisch genannten Verbindungen als neue Stoffe, sowie ein Verfahren zur Herstellung derselben. Dieses Verfahren ist dadurch gekennzeichnet, dass man

a) eine Verbindung der allgemeinen Formel II

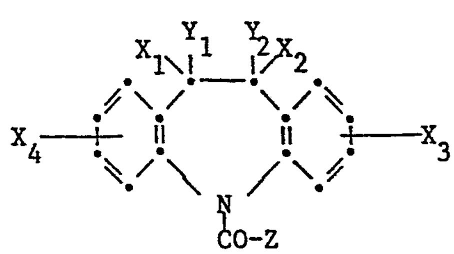

(II)

in welcher Z ein Halogenatom bedeutet, mit einer Verbindung der allgemeinen Formel III

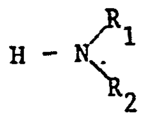

(III)

umsetzt, oder

b) eine Verbindung der allgemeinen Formel IV

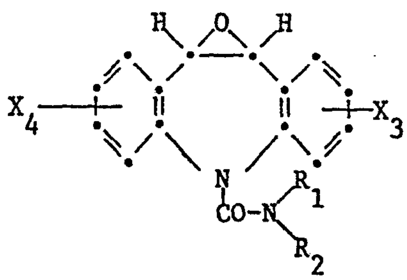

(IV)

in welcher $R_1$, $R_2$, $X_3$ und $X_4$ die unter der Formel I angegebene Bedeutung haben mit einem Niederalkylmercaptan oder einem Alkalimetall-niederalkylmercaptid zu einer Verbindung der allgemeinen Formel I umsetzt, in welcher $X_1$ oder $X_2$ Niederalkylthio, $X_2$ bzw. $X_1$ Wasserstoff und $Y_1$ und $Y_2$ zusammen eine zusätzliche Bindung bedeuten und $R_1$, $R_2$, $X_3$ und $X_4$ die unter der Formel I angegebene Bedeutung haben, umsetzt, oder

c) in einer Verbindung der Formel

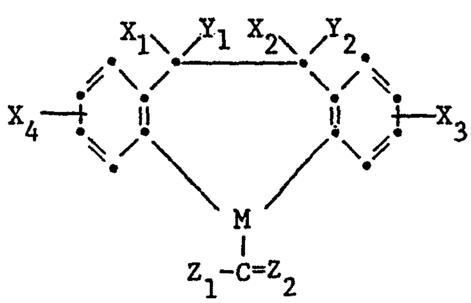

(V)

worin $Z_1$ eine verätherte Mercaptogruppe und $Z_2$ eine Gruppe der Formel n = $NR_1$, $[=NHR_1]^{\oplus}A^{\ominus}$ oder $[=N(R_1)(R_2)]^{\oplus}A^{\ominus}$, in der $A^{\ominus}$ ein Säureanion bedeutet, die $Z_1-C(=Z_2)$-Gruppe zu der entsprechenden $O=C(NR_1R_2)$-Gruppe hydrolysiert und gewünschtenfalls eine Verbindung der allgemeinen Formel I, worin $X_1$ bzw. $X_2$ Niederalkoxy bedeutet und $Y_1$ und $Y_2$

gemeinsam eine zusätzliche Bindung darstellen, zur entsprechenden Verbindung, worin $X_1$ Hydroxy ist, bzw. zur tautomeren Oxoverbindung hydrolysiert und diese gewünschtenfalls zur entsprechenden Verbindung mit Hydroxy $X_1$ und Wasserstoff $Y_1$, $X_2$ und $Y_2$ reduziert oder eine Verbindung der allgemeinen Formel I, in welcher $X_1$, $X_2$ und/oder $X_3$ Niederalkylthio bedeutet, zur entsprechenden Verbindung der allgemeinen Formel I mit Niederalkylsulfonyl oder Niederalkylsulfinyl als $X_1$, $X_2$ und/oder $X_3$ oxidiert.

Die Umsetzung gemäss a) wird vorzugsweise in einem organischen Lösungsmittel, z.B. in einem niederen Alkanol, wie Aethanol, Isopropanol oder Butanol, in einer ätherartigen Flüssigkeit, wie Tetrahydrofuran oder Dioxan, oder einem Kohlenwasserstoff, wie Benzol oder Toluol, bei Raumtemperatur oder vorzugsweise in der Wärme, z.B. bei der Siedetemperatur des verwendeten Lösungsmittels, durchgeführt. Das gegebenenfalls als Ausgangsstoff benötigte Ammoniak und andere gasförmige Ausgangsstoffe der allgemeinen Formel III können am Anfang oder während der ganzen Reaktionsdauer eingeleitet, oder bei Verwendung eines mit Wasser mischbaren Lösungsmittels auch als konzentrierte wässrige Lösung eingesetzt werden. Man kann aber auch flüssiges Ammoniak oder Methylamin verwenden und die Umsetzung nötigenfalls im geschlossenen Gefäss durchführen.

Zu Ausgangsstoffen der allgemeinen Formel II gelangt man beispielsweise durch Umsetzung der entsprechenden 5-unsubstituierten 5H-Dibenz[b,f]azepine mit Phosgen oder Kohlensäuredibromid in einem inerten organischen Lösungsmittel, wie z.B. Benzol oder insbesondere Toluol, bei Raumtemperatur oder mässig erhöhter Temperatur, z.B. bis Siedetemperatur der genannten Lösungsmittel. Von den für diese Umsetzung benötigten 5H-Dibenz[b,f]azepine sind einige bekannt und die weiteren in an sich bekannter Weise herstellbar.

Die gewünschtenfalls an die Umsetzung gemäss a) anschliessende Hydrolyse von Verbindungen mit Niederalkoxy als $X_1$ oder $X_2$ erfolgt gemäss dem Enoläther-Charakter dieser Verbindungen beispielsweise durch

Erwärmen in einer wässrigen oder wässrig organischen Mineralsäure, z.B. in verdünnter z.B. 2-normaler Salzsäure oder verdünnter Schwefelsäure auf mässig erhöhte Temperaturen bis Siedetemperatur, d.h. etwa auf 60 bis etwas über 100°C. Die Hydrolyse kann aber z.B. auch in einem wässrig-organischen Medium, z.B. einem Gemisch von Wasser mit einem Niederalkanol oder Dioxan, in Gegenwart eines sauren Ionenaustauschers, z.B. Amberlite® IR-100 (Markenname der Firma Rohm und Haas, Philadelphia) oder Dowex® (Markenname der Firma Dow Chemical Co., Midland, Michigan) durchgeführt werden.

Die Umsetzung gemäss b) wird vorzugsweise in Gegenwart eines alkalischen Kondensationsmittels, wie eines Alkalimetall-niederalkoxids durchgeführt oder das Niederalkylmercaptan wird in Form eines Alkalimetallmercaptids, z.B. Natriummercaptids, eingesetzt. Die Umsetzung erfolgt z.B. in einem niederen Alkanol,wie Aethanol,bei Raumtemperatur bis mässig erhöhten Temperaturen, z.B. bis 100°C bzw. Siedetemperatur des verwendeten Lösungsmittels. Von den Ausgangsstoffen der allgemeinen Formel IV ist das 1a,10b-Dihydro-6H-dibenz-[b,f]oxireno[d]azepin-6-carboxamid bekannt und weitere sind analog zu dieser Verbindung herstellbar.

Bei der Umsetzung c) werden beispielsweise Verbindungen der Formel V verwendet, in denen die verätherte Mercaptogruppe $Z_1$ aliphatisch veräthertes Mercapto, wie Niederalkylthio, z.B. Methylthio, und das Säureanion $A^{\ominus}$ beispielsweise das Anion einer starken Protonensäure, beispielsweise einer Halogenwasserstoffsäure, wie ein Halogenid-, z.B. das Jodidion, ist. Die Hydrolyse erfolgt vorzugsweise in einem alkalischen Medium, z.B. in einer wässrigen Alkalilauge, wie Natron- oder Kalilauge, vorzugsweise im Temperaturbereich von etwa 60 bis etwa 100°, vorzugsweise in der Siedehitze durchgeführt.

Die Hydrolyse von Niederalkoxy zu Hydroxy erfolgt z.B. durch Behandeln mit einer wässrigen Mineralsäure; die Reduktion der Doppelbindung, der auch andere Verbindungen der Formel I, worin $Y_1$ und $Y_2$ eine Bindung darstellen, zugänglich sind, durch katalytische Hydrierung, z.B. in Gegenwart von Kupferchromit. Die Oxidation von Niederalkylthio zu Niederalkylsulfinyl bzw. -sulfonyl erfolgt beispielsweise mittels Wasserstoffperoxid

in einem gegenüber diesem inerten organischen oder organisch-wässrigen Lösungsmittel, z.B. in gegebenenfalls Wasser enthaltende Essigsäure, bzw. in dem aus Eisessig und wässriger Wasserstoffperoxidlösung entstehenden Gemisch, bei mässig erhöhten Temperaturen zwischen etwa 60 und 100°C, wenn als Oxidationsprodukt eine durch Niederalkylsulfonyl substituierte Verbindung erhalten werden soll. Die Oxidation der Niederalkylthio- zur Niederalkylsulfinylgruppe wird z.B. entweder nach obigem Verfahren im Temperaturbereich von etwa 20 bis 60°C, oder insbesondere unter Verwendung von Alkalimetall-, insbesondere Natrium- oder Kalium-perjodat, wie Natriummetaperjodat in organischwässrigem, z.B. niederalkanolisch-wässrigem, insbesondere äthanolischwässrigem Medium in der Kälte, z.B. bei 0°C bis Raumtemperatur durchgeführt, vgl. hierzu auch GB-PS 1,114,970. Die Ausgangsstoffe für diese Oxidationen können z.B. nach Verfahren a) oder, falls $X_1$ oder $X_2$ Niederalkylthio ist, auch nach Verfahren b) hergestellt werden.

Die Erfindung betrifft auch diejenigen Ausführungsformen des beschriebenen Verfahrens, denen zufolge man von einer auf einer beliebigen Verfahrensstufe als Zwischenprodukt erhältlichen Verbindung ausgeht und die fehlenden Verfahrensschritte ausführt oder einen Ausgangsstoff unter den Reaktionsbedingungen bildet oder in Form eines Derivates einsetzt. Vorzugsweise werden solche Ausgangsstoffe verwendet, die zu den vorstehend hervorgehobenen neuen Verbindungen führen, wobei neue Ausgangsstoffe und Verfahren zu ihrer Herstellung ebenfalls neuer Erfindungsgegenstand darstellen.

In den nachstehenden Beispielen 1 bis 9 wird die erfindungsgemässe Herstellung Verbindungen der allgemeinen Formel I und in den weiteren Beispielen die Herstellung von erfindungsgemässen pharmazeutischen Präparaten näher erläutert, ohne den Umfang der Erfindung in irgendeiner Weise zu beschränken.

Beispiel 1: 27,1 g (0,1 Mol) 3-Methylsulfonyl-5H-dibenz[b,f]azepin werden unter Rühren in eine Lösung von 19,8 g (0,2 Mol) Phosgen in 300 ml abs. Toluol eingetragen. Das Gemisch wird 20 Stunden unter Rückfluss gekocht. Hierauf dampft man es im Rotationsverdampfer vollständig ein, wobei das rohe 3-Methylsulfonyl-5H-dibenz[b,f]azepin-5-carbonylchlorid vom Smp. 160-162° zurückbleibt.

Das obige Rohprodukt wird unter Rühren bei 70° in 600 ml absolutem Aethanol gelöst. In diese Lösung leitet man während 2 Stunden Ammoniakgas ein, wobei die Reaktionslösung immer unter Rückfluss gekocht wird. Anschliessend dampft man das Reaktionsgemisch im Rotationsverdampfer ein, wäscht den Rückstand mit Wasser und kristallisiert das 3-Methylsulfonyl-5H-dibenz[b,f]azepin-5-carboxamid nach dem Trocknen aus Acetonitril um; Smp. 195-197°.

Der Ausgangsstoff kann wie folgt hergestellt werden:

a) Zu einer Lösung von 27,3 g (0,1 Mol) 10,11-Dihydro-3-methylsulfonyl-5H-dibenz[b,f]azepin (vgl. GB-PS 1,000,191) in 100 ml Toluol lässt man unter Rühren innerhalb 30 Minuten 9 g (0,115 Mol) Acetylchlorid zutropfen, wobei die Temperatur zwischen 20 bis 50° gehalten wird. Anschliessend kocht man das Reaktionsgemisch 5 Stunden unter Rückfluss und kühlt es dann auf 5° ab, worauf das 5-Acetyl-10,11-dihydro-3-methylsulfonyl-5H-dibenz[b,f]azepin auskristallisiert. Es wird abgenutscht, mit Wasser gut gewaschen und im Vakuumschrank bei 80° getrocknet, Smp. hierauf 157-159°.

b) 31,5 g (0,1 Mol) 5-Acetyl-10,11-dihydro-3-methylsulfonyl-5H-dibenz-[b,f]azepin werden in 2 Litern Tetrachlorkohlenstoff gelöst und die Lösung mit 17,8 g (0,1 Mol) N-Bromsuccinimid versetzt. Unter Rühren und in einer Stickstoffatmosphäre wird unter Belichtung mit einer UV-Lampe das Gemisch zum Sieden erhitzt. Man hält solange am Sieden, bis sich alles N-Bromsuccinimid, welches auf dem Grund des Gefässes liegt, in auf der Lösung schwimmendes Succinimid umgewandelt hat; was etwa

eine Stunde dauert. Hierauf kühlt man das Reaktionsgemisch auf 20° ab und wäscht es im Scheidetrichter mit Wasser. Nach dem Trocknen über Natriumsulfat wird das Lösungsmittel im Rotationsverdampfer abdestilliert und der ölige Rückstand in 60 ml absolutem Aethanol gelöst. Diese Lösung wird mit einer Lösung von 60 g Kaliumhydroxid in 240 ml abs. Aethanol versetzt und eine Stunde unter Rückfluss gekocht. Nach dem Abkühlen des Reaktionsgemisches auf 20° werden 2000 ml Wasser zugegeben, die ausgefallenen Kristalle des 3-Methylsulfonyl-5H-dibenz[b,f]azepin werden abgenutscht, mit Wasser, dann mit Diäthyläther gewaschen und im Vakuumschrank bei 80° getrocknet; Smp. hierauf 141-143°,

Beispiel 2: Analog Beispiel 1 wird aus 23,9 g (0,1 Mol) 3-Methylthio-5H-dibenz[b,f]azepin und 19,8 g (0,2 Mol) Phosgen in 300 ml abs. Toluol das 3-Methylthio-5H-dibenz[b,f]azepin-5-carbonylchlorid als öliges Rohprodukt und daraus mit Ammoniakgas in 600 ml absolutem Aethanol das 3-Methylthio-5H-dibenz[b,f]azepin-5-carboxamid, Smp. 179-180° (aus Methanol hergestellt.

Das Ausgangsmaterial wird analog Beispiel 1a) und 1b) hergestellt:

a) Aus 24,1 g (0,1 Mol) 10,11-Dihydro-3-methylthio-5H-dibenz[b,f]-azepin (GB-PS 1,114,970) und 9 g (0,115 Mol) Acetylchlorid in 100 ml Toluol erhält man das 5-Acetyl-10,11-dihydro-3-methylthio-5H-dibenz-[b,f]azepin, Smp. 69-74° (aus Petroläther).

b) Aus 28,3 g 5-Acetyl-3-10,11-dihydro-10,11-dihydro-3-methylthio-5H-dibenz[b,f]azepin und 17,8 g (0,1 Mol) N-Bromsuccinimid in 2000 ml Tetrachlorkohlenstoff und anschliessende Behandlung mit 60 g Kaliumhydroxid in 300 ml absolutem Aethanol erhält man das 3-Methylthio-5H-dibenz[b,f]azepin, Smp. 167-169° (aus Aethanol).

Beispiel 3: Analog Beispiel 1 erhält man aus 22,3 g (0,1 Mol) 3-Methoxy-5H-dibenz[b,f]azepin und 19,8 g (0,2 Mol) Phosgen in

in 300 ml absolutem Toluol das 3-Methoxy-5H-dibenz[b,f]azepin-5-carbonylchlorid als öliges Rohprodukt und daraus mit Ammoniakgas in 600 ml absolutem Aethanol das 3-Methoxy-5H-dibenz[b,f]azepin-5-carboxamid, Smp. 154-156° (aus Benzol).

Der Ausgangsstoff wird analog Beispiel 1a) und 1b) aus dem 10,11-Dihydro-3-methoxy-5H-dibenz[b,f]azepin (vgl. GB-PS 926,816) durch Acetylierung, Bromierung und Bromwasserstoffabspaltung hergestellt.

Beispiel 4: Analog Beispiel 1 erhält man aus 30 g (0,1 Mol) 3-(Dimethylsulfamoyl)-5H-dibenz[b,f]azepin (vgl. CH-PS 408,927) und 19,8 g (0,2 Mol) Phosgen in 300 ml absolutem Toluol das 3-(Dimethylsulfamoyl)-5H-dibenz[b,f]azepin-5-carbonylchlorid als öliges Rohprodukt und daraus mit Ammoniakgas in 600 ml absolutem Aethanol das 3-(Dimethylsulfamoyl)-5H-dibenz[b,f]azepin-5-carboxamid, Smp. 215-217° (aus Methanol).

Beispiel 5: Analog Beispiel 1 erhält man aus 21,8 g (0,1 Mol) 5H-Dibenz-[b,f]azepin-3-carbonitril und 19,8 g (0,2 Mol) Phosgen in 300 ml absolutem Toluol das 3-Cyano-5H-dibenz[b,f]azepin-5-carbonylchlorid Smp. 168-170° (Rohprodukt) und daraus mit Ammoniakgas in 600 ml absolutem Aethanol das 3-Cyano-5H-dibenz[b,f]azepin-5-carboxamid, Smp. 269-272° (aus Chloroform).

Der Ausgangsstoff wird wie folgt hergestellt:

a) Analog Beispiel 1a) erhält man aus 27,2 g (0,1 Mol) 3-Brom-10,11-dihydro-5H-dibenz[b,f]azepin und 9 g (0,115 Mol) Acetylchlorid in 300 ml absolutem Toluol das 5-Acetyl-3-brom-10,11-dihydro-5H-dibenz-[b,f]azepin (Rohprodukt), (vgl. US-PS 3,056,774).

b) Analog Beispiel 1b) erhält man aus 31,4 g (0,1 Mol) 5-Acetyl-3-brom-10,11-dihydro-5H-dibenz[b,f]azepin (Rohprodukt) und 17,8 g

(0,1 Mol) N-Bromsuccinimid in 350 ml Tetrachlorkohlenstoff und anschliessender Behandlung mit 60 g Kaliumhydroxid in 300 ml absolutem Aethanol das 3-Brom-5H-dibenz[b,f]azepin, Smp. 218-220° (aus Toluol).

c) 27,2 g (0,1 Mol) 3-Brom-5H-dibenz[b,f]azepin, 10,7 g (0,12 Mol) Kupfercyanid und 50 ml Dimethylformamid werden unter Rühren 4 Stunden unter Rückfluss gekocht. Anschliessend wird das Reaktionsgemisch auf 40° abgekühlt und mit 200 ml einer 50 %igen wässrigen Aethylendiaminlösung und 200 ml Methylenchlorid stark gerührt. Dann wird die organische Phase abgetrennt und die wässrige Phase noch zweimal mit Methylenchlorid extrahiert. Die vereinigten organischen Lösungen werden mit Wasser gewaschen, über Natriumsulfat getrocknet und auf ein kleines Volumen eingeengt, worauf bei Abkühlen das 5H-Dibenz[b,f]azepin-3-carbonitril vom Smp. 181-182° auskristallisiert.

Beispiel 6: Analog Beispiel 1 erhält man aus 21,8 g (0,1 Mol) 5H-Dibenz[b,f]azepin-2-carbonitril und 19,8 g (0,2 Mol) Phosgen in 300 ml absolutem Toluol das 2-Cyano-5H-dibenz[b,f]azepin-5-carbonylchlorid, Smp. 208-209° (Rohrprodukt) und daraus mit Ammoniakgas in 600 ml absolutem Aethanol das 2-Cyano-5H-dibenz[b,f]azepin-5-carboxamid, Smp. ab 284° unter Zersetzung (aus Methylenchlorid).

Der Ausgangsstoff wird wie folgt hergestellt:

a) Analog Beispiel 1a) erhält man aus 27,2 g (0,1 Mol) 2-Brom-10,11-dihydro-5H-dibenz[b,f]azepin und 9 g Acetylchlorid in 300 ml abs. Toluol das 5-Acetyl-2-brom-10,11-dihydro-5H-dibenz[b,f]azepin, Smp. 136-142° (Rohprodukt).

b) Anaolg Beispiel 1b) erhält man aus 31,4 g (0,1 Mol) 5-Acetyl-2-brom-10,11-dihydro-5H-dibenz[b,f]azepin und 17,8 g (0,1 Mol) N-Bromsuccinimid in 350 ml Tetrachlorkohlenstoff und anschliessende Behandlung mit 60 g Kaliumhydroxid in 300 ml absolutem Aethanol das 2-Brom-5H-dibenz[b,f]azepin, Smp. 111-113° (aus Hexan).

c) Analog Beispiel 5c) erhält man aus 27,2 g (0,1 Mol) 2-Brom-5H-dibenz[b,f]azepin, 10,7 g (0,12 Mol) Kupfercyanid und 50 ml Dimethylformamid das 5H-Dibenz[b,f]azepin-2-carbonitril, Smp. 171-173°, (aus Methylenchlorid).

Beispiel 7: 25,6 g (0,1 Mol) 5H-Dibenz[b,f]azepin-5-carbonylchlorid (vgl. GB-PS 906,452) werden unter Rühren bei 70° in 600 ml absolutem Aethanol gelöst. In diese Lösung leitet man während 2 Stunden gasförmiges Methylamin ein, wobei die Reaktionslösung immer unter Rückfluss gekocht wird. Anschliessend dampft man das Reaktionsgemisch im Rotationsverdampfer ein und verrührt den Rückstand mit Wasser. Das Produkt wird abgenutscht, mit Wasser gut nachgewaschen und hierauf im Vakuumschrank bei 70° getrocknet. Nach dem Umkristallisieren aus Aethanol erhält man das N-Methyl-5H-dibenz[b,f]azepin-5-carboxamid, Smp. 210-212°.

In anaolger Weise erhält man unter Verwendung von 27,0 g (0,1 Mol) 10-Methyl-5H-dibenz[b,f]azepin-5-carbonylchlorid (vgl. CH-PS 403,767) das N,10-Dimethyl-5H-dibenz[b,f]azepin-5-carboxamid.

Beispiel 8: 11,5 g (0,5 Mol) Natrium werden in 2000 ml abs. Aethanol unter Rühren gelöst. In diese Lösung werden bei 20-40° 24 g (0,5 Mol) gasförmiges Methylmercaptan eingeleitet. Anschliessend werden 25,1 g (0,1 Mol) 1a,10b-Dihydro-6H-dibenz[b,f]oxireno[d]azepin-6-carboxamid (vgl. DE-OS 2,246,842) zugegeben und das Gemisch 12 Stunden unter Rückfluss gekocht. Hierauf wird das Reaktionsgemisch im Vakuum eingedampft und der Rückstand mit 1500 ml Wasser und 300 ml Diäthyläther 3 Stunden bei 2-5° verrührt. Die Suspension wird genutscht und mit Wasser und Diäthyläther gewaschen. Das feuchte 10-Methylthio-5H-dibenz[b,f]azepin-5-carboxamid wird im Vakuum bei 80° getrocknet und schmilzt nach dem Umkristallisieren aus Methanol bei 175-177°.

Beispiel 9: 28,2 g (0,1 Mol) 10-Methylthio-5H-dibenz[b,f]azepin-5-carboxamid, 100 ml Eisessig und 42 ml 30 %ige wässrige Wasserstoffperoxidlösung werden unter Rühren eine Stunde auf 85° erwärmt. Hierauf kühlt man das Reaktionsgemisch auf 5° ab, wobei das 10-Methylsulfonyl-5H-dibenz[b,f]azepin-5-carboxamid auskristallisiert. Das Produkt wird abgenutscht, mit 2n-Essigsäure, dann mit Wasser und zuletzt mit Aceton gewaschen. Nach dem Trocknen im Vakuumschrank bei 100° schmilzt die Substanz bei 275-278° unter Zersetzung.

Beispiel 10: 26,3 g (0,1 Mol) 10-Trifluormethyl-10,11-dihydro-5H-dibenz[b,f]azepin werden unter Rühren in eine Lösung von 19,8 g (0,2 Mol) Phosgen in 350 ml Toluol eingetragen. Das Gemisch wird unter ständigem Einleiten von Phosgen 20 Stunden zum Rückfluss erhitzt. Danach dampft man am Rotationsverdampfer zur Trockne ein, gibt 50 ml Petroläther/Aether (2:1) hinzu und kühlt unter Rühren auf 5° ab. Das kristallisierte 10-Trifluormethyl-10,11-dihydro-5H-dibenz[b,f]azepin-5-carbonylchlorid wird anschliessend abgenutscht und getrocknet; Smp. 154-158°.

Das obige Produkt wird unter Rühren bei 70° in 400 ml 95 % Aethanol gelöst. In diese Lösung leitet man 5 Stunden lang Ammoniakgas ein, wobei die Reaktionslösung ständig am Rückfluss gehalten wird. Dann dampft man das Reaktionsgemisch am Rotationsverdampfer zur Trockne ein und verrührt den Rückstand mit Wasser und wenig eiskaltem Aether. Das kristallisierte 10-Trifluormethyl-10,11-dihydro-5H-dibenz[b,f]-azepin-5-carboxamid wird abgenutscht, mit wenig Wasser und kaltem Aether gewaschen und getrocknet; Smp. 172-174°.

Das Ausgangsmaterial kann z.B. wie folgt hergestellt werden:

a) 3,3 g (0,1 Mol) 10-Brom-5-acetyl-5H-dibenz[b,f]azepin werden im Autoklaven mit 83,2 g (0,42 Mol) Trifluormethyljodid und 31,6 g (0,5 Mol) Kupferpulver in 250 ml Dimethylformamid 70 Stunden bei 150° gerührt. Dann werden am Rotationsverdampfer 200 ml Dimethylformaid

abdestilliert und der Rückstand 4-mal mit je 800 ml Aether extrahiert. Die vereinigten Aetherextrakte werden 3-mal mit Wasser gewaschen und über Natriumsulfat getrocknet. Nach dem Klären mit Diatomenerde und Kohle wird die Aetherlösung auf 50 ml eingeengt und auf 5° abgekühlt. Das kristallisierte 10-Trifluormethyl-5-acetyl-5H-dibenz[b,f]azepin wird abgenutscht und getrocknet; Smp. 144-147°.

b) 30,3 g (0,1 Mol) 10-Trifluormethyl-5-acetyl-5H-dibenz[b,f]azepin werden in einer Lösung von 30 g Kaliumhydroxid-Plätzchen (85 %) in 120 ml Aethanol unter Rühren $2\frac{1}{2}$ Stunden zum Rückfluss erhitzt. Das Reaktionsgemisch wird dann auf 900 ml Wasser gegossen und 3-mal mit je 300 ml Aether extrahiert. Die vereinigten Aetherextrakte werden zweimal mit Wasser gewaschen und dann über Natriumsulfat getrocknet. Das Lösungsmittel wird im Rotationsverdampfer abdestilliert. Der ölige Rückstand wird in 90 ml Hexan und 10 ml Aether gelöst. Das 10-Trifluormethyl-5H-dibenz[b,f]azepin kristalliert beim Abkühlen auf 20° und wird abgenutscht und getrocknet; Smp. 123-126°.

c) 26,1 g (0,1 Mol) 10-Trifluormethyl-5H-dibenz[b,f]azepin werden in 500 ml Essigester mit 4,4 g 10 % Pd/C bei 20-22° bei Normaldruck 40 Stunden lang hydriert. Dann wird der Katalysator abfiltriert und das Filtrat im Rotationsverdampfer eingedampft. Den öligen Rückstand reinigt man durch Chromatographie an 400 g Kieselgel. Das 10-Trifluormethyl-10,11-dihydro-5H-dibenz[b,f]azepin wird mit Petrolaether/Toluol (4:1) eluiert; Smp. 57-59.

Beispiel 11: Analog Beispiel 1 wird aus 26,1 g (0,1 Mol) 10-Trifluormethyl-5H-dibenz[b,f]azepin und 19,8 g (0,2 Mol) Phosgen in 350 ml Toluol das 10-Trifluor-5H-dibenz[b,f]azepin-5-carbonylchlorid hergestellt; Smp. 140-144°. Dieses wird ebenfalls nach Beispiel 1 mit Ammoniakgas in 400 ml Aethanol in das vom 10-Trifluormethyl-5H-dibenz[b,f]azepin-5-carboxamid überführt; Smp. 194-196°.

Beispiel 12: 28,2 g (0,1 Mol) 10-Methylthio-5H-dibenz[b,f]azepin-5-carboxamid werden mit 130 ml 30 % Wasserstoffperoxid in 600 ml Aethanol 3 Stunden zum Rückfluss erhitzt. Dann dampft man das Reaktionsgemisch im Rotationsverdampfer zur Trockne ein. Der Rückstand wird in 100 ml Aceton gelöst und mit 500 ml absolutem Aether versetzt. Das 10-Methylsulfinyl-5H-dibenz[b,f]azepin-5-carboxamid wird abgenutscht und getrocknet; Smp. 242-247°.

Beispiel 13: In analoger Weise wie in den Beispielen 1-12 beschrieben kann man ferner herstellen:

10-Methoxy-11-methyl-5H-dibenz[b,f]azepin-5-carboxamid,
10-Cyano-11-methoxy-5H-dibenz[b,f]azepin-5-carboxamid,
10-Brom-5H-dibenz[b,f]azepin-5(N-methyl)carboxamid,
10-Cyano-5H-dibenz[b,f]azepin-5(N-methyl)carboxamid,
3,7-Dichlor-10,11-dihydro-5H-dibenz[b,f]azepin-5-carboxamid,
3,7-Dimethyl-10,11-dihydro-5H-dibenz[b,f]azepin-5-carboxamid,
2,8-Dimethyl-10,11-dihydro-5H-dibenz[b,f]azepin-5-carboxamid,
3-Methylthio-10,11-dihydro-5H-dibenz[b,f]azepin-5-carboxamid,
3-Dimethylsulfamoyl-10,11-dihydro-5H-dibenz[b,f]azepin-5-carboxamid,
5-[(1-Piperidinyl)-carbonyl]-10,11-dihydro-10-hydroxy-5H-dibenz[b,f]-azepin,
5-[(1-Piperidinyl)-carbonyl]-10,11-dihydro-10-oxo-5H-dibenz[b,f]-azepin,
3-Methylsulfonyl-10,11-dihydro-5H-dibenz[b,f]azepin-5-carboxamid.

Beispiel 14: Tabletten, enthaltend je 25 mg 3-Methylsulfonyl-5H-dibenz[b,f]azepin-5-carboxamid, können wie folgt hergestellt werden:

Zusammensetzung (für 1000 Tabletten)

| | |
|---|---|
| 3-Methylsulfonyl-5H-dibenz[b,f]azepin-5-carboxamid | 25,0 g |
| Lactose | 250,0 g |
| Kartoffelstärke | 176,0 g |
| Gelatine | 4,0 g |
| Talk | 31,0 g |
| Magnesiumstearat | 5,0 g |
| Siliciumdioxid (hochdispers) | 10,0 g |
| Aethanol | q.s. |

Der Wirkstoff wird mit der Lactose und 146 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Silicumdioxid zu und presst das Gemisch zu Tabletten von je 73,0 mg Gewicht und dem oben angegebenen Wirkstoffgehalt, die gewünschtenfalls mit Teilkerben zur feineren Anpassung der Dosierung versehen sein können.

Anstelle des obengenannten Wirkstoffes kann z.B. auch die gleiche Menge 10-Methylthio-5H-dibenz[b,f]azepin-5-carboxamid verwendet werden.

In analoger Weise kann man auch Tabletten enthaltend eine andere der in den Beispielen bzw. spezifisch in der Beschreibung genannten Verbindung der allgemeinen Formel I herstellen.

Patentansprüche für die Vertragsstaaten: CH, LI, DE, FR, LU, NL, SE

1. Neue 5H-Dibenz[b,f]azepin-5-carboxamide der allgemeinen Formel I

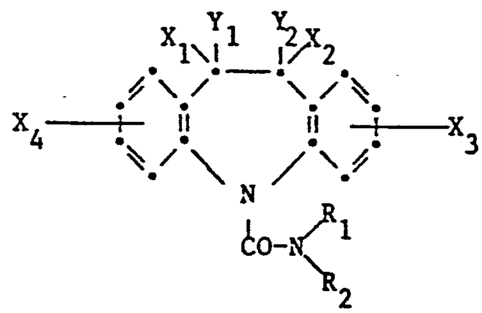

(I),

worin $R_1$ und $R_2$ unabhängig voneinander Wasserstoff oder Niederalkyl oder unter sich verbunden Niederalkylen oder Aethylenoxyäthylen darstellen, eines der Symbole $X_1$ und $X_2$ Niederalkylthio, Niederalkylsulfinyl, Niederalkylsulfonyl, Trifluormethyl, Niederalkoxy, Halogen der Atomnummer bis und mit 35 oder Cyano und das andere Wasserstoff, Niederalkyl, Niederalkoxy, Niederalkylthio, Niederalkylsulfinyl, Niederlakylsulfonyl oder Trifluormethyl bedeutet oder beide Symbole $X_1$ und $X_2$ für Wasserstoff stehen, $X_3$ Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl darstellt oder, sofern mindestens einer der Reste $X_1$ und $X_2$ von Wasserstoff verschieden ist und zugleich mit Halogen oder Cyano als $X_1$ bzw. $X_2$ und Wasserstoff als $X_2$ bzw. $X_1$ $R_1$ Wasserstoff oder Niederalkyl und $R_2$ Niederalkyl oder $R_1$ gemeinsam mit $R_2$ Niederalkylen oder Aethylenoxyäthylen und zugleich mit Niederalkoxy als $X_1$ bzw. $X_2$ und Wasserstoff als $X_2$ bzw. $X_1$ $R_1$ gemeinsam mit $R_2$ Niederalkylen oder Aethylenoxyäthylen bedeutet, auch Wasserstoff bedeuten kann, $X_4$ Wasserstoff bedeutet oder zugleich mit Niederalkyl oder Halogen als $X_3$ ebenfalls einen dieser Reste darstellen kann, und die Symbole $Y_1$ und $Y_2$, sofern eines der Symbole $X_1$ und $X_2$ Niederalkylthio, Niederalkylsulfinyl, Niederalkylsulfonyl oder Trifluormethyl und das andere Wasserstoff bedeutet oder beide Wasserstoff darstellen, jeweils für Wasserstoff stehen oder, sofern mindestens einer der Reste $R_1$, $R_2$, $X_1$ und $X_2$ von Wasserstoff oder $X_3$ von Cyano oder mindestens einer der Reste $X_3$ und $X_4$ von Halogen verschieden ist, gemeinsam eine zusätzliche Bindung darstellen, oder worin $R_1$ und $R_2$ gemeinsam für Niederalkylen oder Aethylenoxyäthylen stehen und $X_1$ gemeinsam mit $Y_1$ Oxo und $X_2$ sowie $Y_2$ Wasserstoff bzw. $X_1$ Hydroxy und $X_2$, $Y_1$ sowie $Y_2$ Wasserstoff darstellt oder $R_1$ und $R_2$ unabhängig voneinander Niederalkyl oder gemeinsam Niederalkylen oder Aethylenoxyäthylen bedeuten, $X_1$ gemeinsam mit $X_2$ Epoxy oder Epimethylen und $Y_1$ sowie $Y_2$ jeweils Wasserstoff darstellt, $X_3$ für Wasserstoff, Niederalkyl, Niederalkoxy, Niederlakylthio, Niederalkylsulfinyl, Niederalkylsulfonyl, Trifluormethyl, Halogen der Atomnummer bis und mit 35, Cyano oder Diniederalkylsulfamoyl steht, und $X_4$ Wasserstoff bedeutet oder zugleich mit Niederalkyl oder Halogen als $X_3$ ebenfalls einen dieser Reste darstellt.

2. Neue 5H-Dibenz[b,f]azepin-5-carboxamide der allgemeinen Formel I

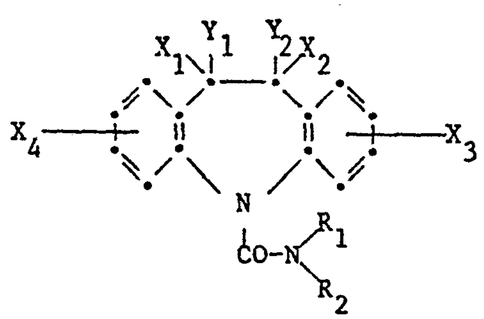

(I),

worin $R_1$ und $R_2$ unabhängig voneinander Wasserstoff oder Niederalkyl oder unter sich verbunden Niederalkylen oder Aethylenoxyäthylen, eines der beiden Symbole $X_1$ und $X_2$ und/oder das Symbol $X_3$ Niederalkylthio, Niederalkylsulfinyl, Niederalkylsulfonyl oder Trifluormethyl und $X_3$ auch Halogen bis Atomnummer 35, Diniederalkylsulfamoyl, Niederalkyl, Cyano oder Niederalkoxy bedeutet und das bzw. die beiden restlichen der Symbole $X_1$, $X_2$ und $X_3$ Wasserstoff bedeuten, jedoch $X_1$ oder $X_2$, sofern mindestens einer der Reste $R_1$ und $R_2$ von Wasserstoff und Niederalkyl oder einer der Reste $X_3$ und $X_4$ von Wasserstoff verschieden ist und $Y_1$ und $Y_2$ eine zusätzliche Bindung darstellen, auch Niederalkoxy und $X_2$ bzw. $X_1$ Wasserstoff bedeuten kann, $X_4$ Wasserstoff bedeutet oder zugleich mit Halogen oder Niederalkyl als $X_3$ ebenfalls einen dieser Reste bedeuten kann, $Y_1$ und $Y_2$ zusammen eine zusätzliche Bindung bedeuten oder zugleich mit Wasserstoff als $X_1$ und $X_2$ auch je Wasserstoff bedeuten können, oder falls mindestens einer der Reste $R_1$ und $R_2$ von Wasser-

stoff verschieden ist und $X_3$ und $X_4$ Wasserstoff bedeuten, $X_1$ auch Halogen bis Atomnummer 35 oder Cyano, $X_2$ Wasserstoff und $Y_1$ und $Y_2$ zusammen eine zusätzliche Bindung bedeuten können.

3. Verbindungen gemäss Anspruch 2, mit der Massgabe, dass in Verbindungen der Formel I, worin $X_1$ und $X_2$ für Wasserstoff und $Y_1$ sowie $Y_2$ gemeinsam für eine zusätzliche Bindung stehen, $X_3$ von Cyano verschieden ist, wenn $R_1$ und $R_2$ für Wasserstoff stehen und $X_4$ von Halogen verschieden ist, wenn $X_3$ Halogen ist.

4. Verbindungen gemäss Anspruch 1, worin $R_1$ und $R_2$ unabhängig voneinander Wasserstoff oder Niederalkyl oder gemeinsam Niederalkylen oder Aethylenoxyäthylen darstellen, eines der Symbole $X_1$ und $X_2$ Niederalkoxy, Niederalkylthio, Niederalkylsulfinyl, Niederalkylsulfonyl, Trifluormethyl oder, sofern mindestens einer der Reste $R_1$ und $R_2$ von Wasserstoff verschieden ist, Halogen der Atomnummer bis und mit 35 oder Cyano und das andere Wasserstoff bedeutet oder beide Wasserstoff oder unabhängig voneinander Niederalkylthio, Niederalkylsulfinyl, Niederalkylsulfonyl oder Trifluormethyl darstellen, das Symbol $X_3$, sofern $X_1$ bzw. $X_2$ für Halogen oder Cyano oder zugleich mit Niederalkylen oder Aethylenoxyäthylen als $R_1$ und $R_2$ für Niederalkoxy und $X_2$ bzw. $X_1$ für Wasserstoff steht oder $X_1$ und $X_2$ unabhängig voneinander Niederalkylthio, Niederalkylsulfinyl, Niederalkylsulfonyl oder Trifluormethyl bedeuten, Wasserstoff oder, sofern eines der Symbole $X_1$ und $X_2$ Wasserstoff und das andere Niederalkoxy, Niederalkylthio, Niederalkylsulfinyl, Niederalkylsulfonyl oder Trifluormethyl bedeutet oder beide Symbole $X_1$ und $X_2$ Wasserstoff oder unabhängig voneinander Niederalkylthio, Niederalkylsulfinyl, Niederalkylsulfonyl oder Trifluormethyl bedeuten, Niederalkyl, Niederalkoxy, Niederalkylthio, Niederalkylsulfinyl, Niederalkylsulfonyl, Diniederalkylsulfamoyl, Halogen der Atomnummer bis und mit 35 oder, sofern mindestens einer der Reste $R_1$, $R_2$, $X_1$ und $X_2$ von Wasserstoff verschieden ist, Cyano und $X_4$ Wasserstoff darstellt oder einer der Reste $X_3$ und $X_4$ Niederalkyl oder Halogen und der andere Niederalkyl bedeutet oder, sofern mindestens einer der Reste $X_1$ und $X_2$ von Wasser-

stoff verschieden ist, beide Halogen darstellen und die Symbole $Y_1$ und $Y_2$ gemeinsam eine zusätzliche Bindung oder, sofern $X_1$ und $X_2$ beide für Wasserstoff stehen, ebenfalls jeweils Wasserstoff darstellen.

5. Verbindungen gemäss Anspruch 1, worin $R_1$ und $R_2$ unabhängig voneinander Wasserstoff oder Niederalkyl oder unter sich verbunden Niederalkylen oder Aethylenoxyäthylen bedeuten, eines der beiden Symbole $X_1$ und $X_2$ Niederalkylthio, Niederalkylsulfinyl, Niederalkylsulfonyl oder Trifluormethyl und das andere Wasserstoff darstellt, $X_3$ Wasserstoff, Niederalkylthio, Niederalkylsulfinyl, Niederalkylsulfonyl, Trifluormethyl, Cyano, Halogen bis Atomnummer 35, Diniederalkylsulfamoyl, Niederalkyl oder Niederalkoxy bedeutet, $X_4$ Wasserstoff bedeutet oder zugleich mit Halogen oder Niederalkyl als $X_3$ ebenfalls einen dieser Reste bedeuten kann und $Y_1$ und $Y_2$ zusammen eine zusätzliche Bindung oder jeweils Wasserstoff darstellen.

6. Verbindungen gemäss einem der Ansprüche 1 bis 4, worin $R_1$ und $R_2$ unabhängig voneinander Wasserstoff oder Niederalkyl oder unter sich verbunden Niederalkylen oder Aethylenoxyäthylen, $X_1$ und $X_2$ Wasserstoff bedeuten, $X_3$ Halogen der Atomnummer bis und mit 35, Niederalkyl, Niederalkoxy, Niederalkylthio, Niederalkylsulfinyl, Niederalkylsulfonyl, Trifluormethyl, Cyano oder Diniederalkylsulfamoyl bedeutet, $X_4$ Wasserstoff bedeutet oder zugleich mit Halogen oder Niederalkyl als $X_3$ ebenfalls einen dieser Reste bedeuten kann und $Y_1$ und $Y_2$ jeweils Wasserstoff oder, sofern mindestens einer der Reste $R_1$ und $R_2$ von Wasserstoff oder $X_3$ von Cyano oder $X_4$ von Halogen verschieden ist, zusammen eine zusätzliche Bindung darstellen.

7. Verbindungen gemäss einem der Ansprüche 1, 2 und 4, worin eines der Symbole $R_1$ und $R_2$ Niederalkyl und das andere Wasserstoff bedeutet oder beide für Niederalkyl oder gemeinsam für Niederalkylen oder Aethylenoxyäthylen stehen, eines der Symbole $X_1$ und $X_2$ Halogen der Atomnummer bis und mit 35 oder Cyano und das andere ebenso wie $X_3$ und $X_4$ für Wasserstoff steht, wobei $Y_1$ und $Y_2$ gemeinsam eine zusätzliche Bindung darstellen.

8. Verbindungen gemäss Anspruch 1, worin $R_1$ und $R_2$ unabhängig voneinander Wasserstoff oder Niederalkyl oder unter sich verbunden Niederalkylen oder Aethylenoxyäthylen darstellen, $X_1$ und $Y_1$ gemeinsam Oxo und $X_2$ und $Y_2$ jeweils Wasserstoff bzw. $X_1$ Niederalkoxy oder Hydroxy und $X_2$, $Y_1$ und $Y_2$ jeweils Wasserstoff darstellen, $X_3$ für Halogen der Atomnummer bis und mit 35, Niederalkyl, Niederalkoxy, ferner Niederalkylthio, Niederalkylsulfinyl, Niederalkylsulfonyl, Trifluormethyl, Cyano oder Diniederalkylsulfamoyl oder, sofern $R_1$ und $R_2$ gemeinsam Niederalkylen oder Aethylenoxyäthylen darstellen, für Wasserstoff steht und $X_4$ Wasserstoff bedeutet oder zugleich mit Halogen oder Niederalkyl als $X_3$ ebenfalls einen dieser Reste bedeuten kann.

9. Verbindungen gemäss einem der Ansprüche 1, 2 und 4, worin $R_1$, $R_2$, $X_1$ und $X_2$ Wasserstoff bedeuten, $X_3$ Niederalkylthio, Niederalkylsulfinyl oder Niederalkylsulfonyl mit jeweils bis und mit 4 C-Atomen, Diniederalkylsulfamoyl mit bis und mit 4 C-ATomen in jedem Alkylteil, Niederalkyl oder Niederalkoxy mit bis und mit 4 C-Atomen, Halogen der Atomnummer bis und mit 35 oder Trifluormethyl bedeutet, $X_4$ für Wasserstoff steht oder zugleich mit Niederalkyl oder Halogen als $X_3$ auch für Niederalkyl stehen kann und $Y_1$ und $Y_2$ jeweils Wasserstoff oder zusammen eine zusätzliche Bindung darstellen.

10. Verbindungen gemäss Anspruch 1, worin $R_1$ und $R_2$ unabhängig voneinander Wasserstoff oder Niederalkyl darstellen, welches je bis und mit 4 C-Atome aufweist, einer der Reste $X_1$ und $X_2$ Niederalkylthio, Niederalkylsulfinyl oder Niederalkylsulfonyl mit jeweils bis und mit 4 C-Atomen oder Trifluormethyl oder, sofern mindestens einer der Reste $R_1$ und $R_2$ Niederalkyl darstelle, Halogen der Atomnummer bis und mit 35 oder Cyano bedeutet und der andere sowie $X_3$ und $X_4$ Wasserstoff ist und $Y_1$ und $Y_2$ zusammen eine zusätzliche Bindung darstellen.

11. Verbindungen gemäss einem der Ansprüche 1, 2 und 4, worin 5 der Symbole $R_1$, $R_2$, $X_1$, $X_2$, $X_3$ und $X_4$ Wasserstoff darstellen und ein davon verschiedener Rest $R_1$ oder $R_2$ Niederalkyl mit bis und mit 4 C-Atomen, ein davon verschiedener

Rest $X_1$ oder $X_2$ Niederalkylthio, Niederalkylsulfinyl oder Niederalkylsulfonyl mit jeweils bis und mit 4 C-Atomen oder Trifluormethyl bzw. ein davon verschiedener Rest $X_3$ Niederalkylthio, Niederalkylsulfinyl oder Niederalkylsulfonyl mit jeweils bis und mit 4 C-Atomen, Diniederalkylsulfamoyl mit bis und mit 4 C-Atomen in jedem Alkylteil, Niederalkyl oder Niederalkoxy mit bis und mit 4 C-Atomen, Halogen der Atomnummer bis und mit 35 oder Trifluormethyl bedeutet und $Y_1$ und $Y_2$ zusammen eine zusätzliche Bindung darstellen.

12. 3-Methylsulfonyl-5H-dibenz[b,f]azepin-5-carboxamid,
3-Methylthio-5H-dibenz[b,f]azepin-5-carboxamid,
3-Methoxy-5H-dibenz[b,f]azepin-5-carboxamid ,
3-(Dimethylsulfamoyl)-5H-dibenz[b,f]azepin-5-carboxamid ,
2-Cyano-5H-dibenz[b,f]azepin-5-carboxamid,
N-Methyl-5H-dibenz[b,f]azepin-5-carboxamid,
N-10-Dimethyl-5H-dibenz[b,f]azepin-5-carboxamid ,
10-Methylthio-5H-dibenz[b,f]azepin-5-carboxamid und/oder
10-Methylsulfonyl-5H-dibenz[b,f]azepin-5-carboxamid.

13. 10-Trifluormethyl-5H-dibenz[b,f]azepin-5-carboxamid,
10-Trifluormethyl-10,11-dihydro-5H-dibenz[b,f]azepin-5-carboxamid,
10-Methoxy-11-methyl-5H-dibenz[b,f]azepin-5-carboxamid,
10-Cyano-5H-dibenz[b,f]azepin-5-(N-methyl)-carboxamid,
3,7-Dichlor-10,11-dihydro-5H-dibenz[b,f]azepin-5-carboxamid,
3,7-Dimethyl-10,11-dihydro-5H-dibenz[b,f]azepin-5-carboxamid,
2,8-Dimethyl-10,11-dihydro-5H-dibenz[b,f]azepin-5-carboxamid,
3-Methylthio-10,11-dihydro-5H-dibenz[b,f]azepin-5-carboxamid,
3-Dimethylsulfamoyl-10,11-dihydro-5H-dibenz[b,f]azepin-5-carboxamid,
10-Methylsulfinyl-5H-dibenz[b,f]azepin-5-carboxamid,
3-Chlor-5H-dibenz[b,f]azepin-5-carboxamid,

5-[(1-Piperidinyl)-carbonyl]-10,11-dihydro-10-oxo-5H-dibenz[b,f]-azepin,

5-[(1-Piperidinyl)-carbonyl]-10,11-dihydro-10-hydroxy-5H-dibenz-[b,f]azepin,

3-Methylsulfonyl-10,11-dihydro-5H-dibenz[b,f]azepin-5-carboxamid und/oder

10-Cyano-11-methoxy-5H-dibenz[b,f]azepin-5-carboxamid.

14. 5H-Dibenz[b,f]azepin-5-carboxamide der allgemeinen Formel I

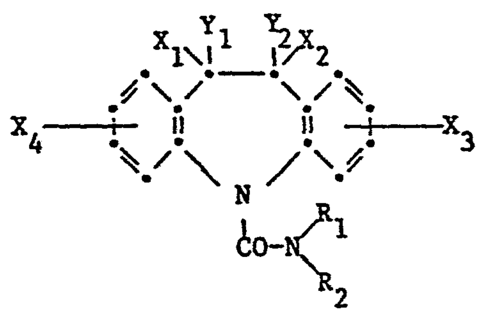

(I)

worin $R_1$ und $R_2$ unabhängig voneinander Wasserstoff oder Niederalkyl oder unter sich verbunden Niederalkylen oder Aethylenoxyäthylen, darstellen, eines der Symbole $X_1$ und $X_2$ Niederalkylthio, Niederalkylsulfinyl, Niederalkylsulfonyl, Trifluormethyl, Niederalkoxy, Halogen der Atomnummer bis und mit 35 oder Cyano und das andere Wasserstoff, Niederalkyl, Niederalkoxy, Niederalkylthio, Niederalkylsulfinyl, Niederalkylsulfonyl oder Trifluormethyl bedeutet oder beide Symbole $X_1$ und $X_2$ für Wasserstoff stehen, $X_3$ Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl darstellt oder, sofern mindestens einer der Reste $X_1$ und $X_2$ von Wasserstoff verschieden ist und zugleich mit Halogen oder Cyano als $X_1$ bzw. $X_2$ und Wasserstoff als $X_2$ bzw. $X_1$ $R_1$ Wasserstoff oder Niederalkyl und $R_2$ Niederalkyl oder $R_1$ gemeinsam mit $R_2$ Niederalkylen oder Aethylenoxyäthylen bedeutet, auch Wasserstoff bedeuten kann, $X_4$ Wasserstoff bedeutet oder zugleich mit Niederalkyl oder Halogen als $X_3$ ebenfalls einen dieser Reste darstellen kann,

und die Symbole $Y_1$ und $Y_2$, sofern eines der Symbole $X_1$ und $X_2$ Niederalkylthio, Niederalkylsulfinyl, Niederalkylsulfonyl oder Trifluormethyl und das andere Wasserstoff bedeutet oder beide Wasserstoff darstellen, jeweils für Wasserstoff stehen oder, sofern mindestens einer der Reste $R_1$, $R_2$, $X_1$ und $X_2$ von Wasserstoff oder $X_3$ von Cyano oder mindestens einer der Reste $X_3$ und $X_4$ von Halogen verschieden ist, gemeinsam eine zusätzliche Bindung darstellen, oder worin $R_1$ und $R_2$ gemeinsam für Niederalkylen oder Aethylenoxyäthylen stehen und $X_1$ gemeinsam mit $Y_1$ Oxo und $X_2$ sowie $Y_2$ Wasserstoff bzw. $X_1$ Hydroxy und $X_2$, $Y_1$ und $Y_2$ Wasserstoff darstellt oder $R_1$ und $R_2$ unabhängig voneinander Niederalkyl oder gemeinsam Niederalkylen oder Aethylenoxyäthylen bedeuten, $X_1$ gemeinsam mit $X_2$ Epoxy oder Epimethylen und $Y_1$ sowie $Y_2$ jeweils Wasserstoff darstellt, $X_3$ für Wasserstoff, Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl steht und $X_4$ Wasserstoff bedeutet oder zugleich mit Niederalkyl oder Halogen als $X_3$ ebenfalls einen dieser Reste darstellt, zur Anwendung in einem Verfahren zur therapeutischen Behandlung des menschlichen oder tierischen Körpers.

15. Verbindungen gemäss Anspruch 14, worin $R_1$ und $R_2$ unabhängig voneinander Wasserstoff oder Niederalkyl oder gemeinsam Niederalkylen oder Aethylenoxyäthylen darstellen, eines der Symbole $X_1$ und $X_2$ Niederalkoxy, Niederalkylthio, Niederalkylsulfinyl, Niederalkylsulfonyl, Trifluormethyl oder, sofern mindestens einer der Reste $R_1$ und $R_2$ von Wasserstoff verschieden ist, Halogen der Atomnummer bis und mit 35 oder Cyano und das andere Wasserstoff bedeutet oder beide Wasserstoff oder unabhängig voneinander Niederalkylthio, Niederalkylsulfinyl, Niederalkylsulfonyl oder Trifluormethyl darstellen, das Symbol $X_3$, sofern $X_1$ bzw. $X_2$ für Halogen oder Cyano oder für Niederalkoxy und $X_2$ bzw. $X_1$ für Wasserstoff steht oder $X_1$ und $X_2$ unabhängig voneinander Niederalkylthio, Niederalkylsulfinyl, Niederalkylsulfonyl oder

Trifluormethyl bedeuten, Wasserstoff oder, sofern eines der Symbole $X_1$ und $X_2$ Wasserstoff und das andere Niederalkoxy, Niederalkylthio, Niederalkylsulfinyl, Niederalkylsulfonyl oder Trifluormethyl bedeutet oder beide Symbole $X_1$ und $X_2$ Wasserstoff oder unabhängig voneinander Niederalkylthio, Niederalkylsulfinyl, Niederalkylsulfonyl oder Trifluormethyl bedeuten, Niederalkyl, Niederalkoxy, Niederalkylthio, Niederalkylsulfinyl, Niederalkylsulfonyl, Diniederalkylsulfamoyl, Halogen der Atomnummer bis und mit 35 oder, sofern mindestens einer der Reste $R_1$, $R_2$, $X_1$ und $X_2$ von Wasserstoff verschieden ist, Cyano und $X_4$ Wasserstoff darstellt oder einer der Reste $X_3$ und $X_4$ Niederalkyl oder Halogen und der andere Niederalkyl bedeutet oder, sofern mindestens einer der Reste $X_1$ und $X_2$ von Wasserstoff verschieden ist, beide Halogen darstellen und die Symbole $Y_1$ und $Y_2$ gemeinsam eine zusätzliche Bindung oder, sofern $X_1$ und $X_2$ beide für Wasserstoff stehen, ebenfalls jeweils Wasserstoff darstellen zur Anwendung gemäss Anspruch 14.

16. Verbindungen gemäss Anspruch 14, worin $X_1$ Niederalkoxy bedeutet, $R_1$ und $R_2$ Wasserstoff oder Niederalkyl, $X_2$, $X_3$ und $X_4$ je Wasserstoff und $Y_1$ und $Y_2$ zusammen eine zusätzliche Bindung bedeuten, oder eine Verbindung gemäss einem der Ansprüche 1 bis 13 zur Anwendung gemäss Anspruch 14, z.B. als Nootropikum.

17. 10-Methoxy-5H-dibenz[b,f]azepin-5-carboxamid,
N-Methyl-10-methoxy-5H-dibenz[b,f]azepin-5-carboxamid,
N,N-Dimethyl-10-methoxy-5H-dibenz[b,f]azepin-5-carboxamid,
N-Butyl-10-methoxy-5H-dibenz[b,f]azepin-5-carboxamid oder
N,N-Dibutyl-10-methoxy-5H-dibenz[b,f]azepin-5-carboxamid zur Anwendung gemäss Anspruch 14.

18. Pharmazeutische Präparate enthaltend eine Verbindung gemäss einem der Ansprüche 2 bis 4, 6 bis 8, 12 und 15 bis 17 neben üblichen pharmazeutischen Hilfs- und/oder Trägerstoffen.

19. Pharmazeutische Präparate enthaltend eine Verbindung gemäss einem der Ansprüche 1, 3, 5, 9 bis 11, 13 und 14 neben üblichen pharmazeutischen Hilfs- und/oder Trägerstoffen.

20. Verfahren zur Herstellung neuer 5H-Dibenz[b,f]azepin-5-carboxamide der allgemeinen Formel I

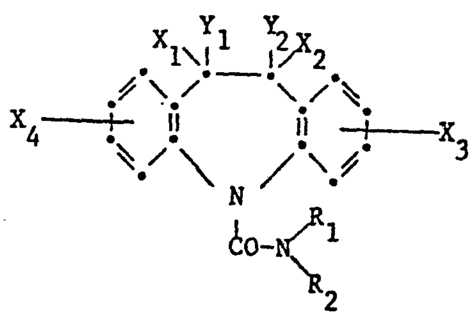

(I),

worin $R_1$ und $R_2$ unabhängig voneinander Wasserstoff oder Niederalkyl oder unter sich verbunden Niederalkylen oder Aethylenoxyäthylen, darstellen, eines der Symbole $X_1$ und $X_2$ Niederalkylthio, Niederalkylsulfinyl, Niederalkylsulfonyl, Trifluormethyl, Niederalkoxy, Halogen der Atomnummer bis und mit 35 oder Cyano und das andere Wasserstoff, Niederalkyl, Niederalkoxy, Niederalkylthio, Niederalkylsulfinyl, Niederalkylsulfonyl oder Trifluormethyl bedeutet oder beide Symbole $X_1$ und $X_2$ für Wasserstoff stehen, $X_3$ Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl darstellt oder, sofern mindestens einer der Reste $X_1$ und $X_2$ von Wasserstoff verschieden ist und zugleich mit Halogen oder Cyano als $X_1$ bzw. $X_2$ und Wasserstoff als $X_2$ bzw. $X_1$ $R_1$ Wasserstoff oder Niederalkyl und $R_2$ Niederalkyl oder $R_1$ gemeinsam mit $R_2$ Niederalkylen oder Aethylenoxyäthylen und zugleich mit Niederalkoxy als $X_1$ bzw. $X_2$ und Wasserstoff als $X_2$ bzw. $X_1$ $R_1$ gemeinsam mit $R_2$ Niederalkylen oder Aethylenoxyäthylen bedeutet, auch Wasserstoff bedeuten kann, $X_4$ Wasserstoff bedeutet oder zugleich mit Niederalkyl oder Halogen als $X_3$ ebenfalls einen dieser Reste darstellen kann, und die

Symbole $Y_1$ und $Y_2$, sofern eines der Symbole $X_1$ und $X_2$ Niederalkylthio, Niederalkylsulfinyl, Niederalkylsulfonyl oder Trifluormethyl und das andere Wasserstoff bedeutet oder beide Wasserstoff darstellen, jeweils für Wasserstoff stehen oder, sofern mindestens einer der Reste $R_1$, $R_2$, $X_1$ und $X_2$ von Wasserstoff oder $X_3$ von Cyano oder mindestens einer der Reste $X_3$ und $X_4$ von Halogen verschieden ist, gemeinsam eine zusätzliche Bindung darstellen, oder worin $R_1$ und $R_2$ gemeinsam für Niederalkylen oder Aethylenoxyäthylen stehen und $X_1$ gemeinsam mit $Y_1$ Oxo und $X_2$ sowie $Y_2$ Wasserstoff bzw. $X_1$ Hydroxy und $X_2$, $Y_1$ sowie $Y_2$ Wasserstoff darstellt oder $R_1$ und $R_2$ unabhängig voneinander Niederalkyl oder gemeinsam Niederalkylen oder Aethylenoxyäthylen bedeuten, $X_1$ oder gemeinsam mit $X_2$ Epoxy oder Epimethylen und $Y_1$ sowie $Y_2$ jeweils Wasserstoff darstellt, $X_3$ für Wasserstoff, Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl steht, und $X_4$ Wasserstoff bedeutet oder zugleich mit Niederalkyl oder Halogen als $X_3$ ebenfalls einen dieser Reste darstellt, dadurch gekennzeichnet, dass man

a) eine Verbindung der allgemeinen Formel II

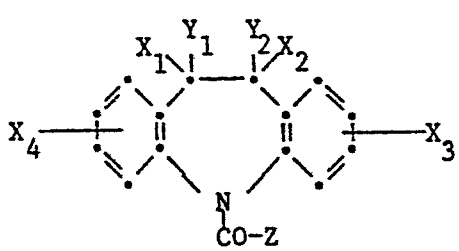

(II)

in welcher Z ein Halogenatom bedeutet, mit einer Verbindung der allgemeinen Formel III

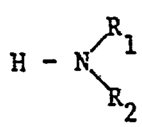

(III)

umsetzt, oder

b) eine Verbindung der allgemeinen Formel IV

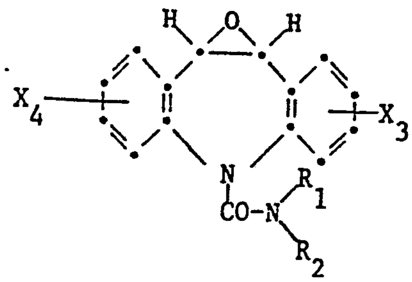

(IV)

in welcher $R_1$, $R_2$, $X_3$ und $X_4$ die unter der Formel I angegebene Bedeutung haben mit einem Niederalkylmercaptan oder einem Alkalimetall-niederalkylmercaptid zu einer Verbindung der allgemeinen Formel I umsetzt, in welcher $X_1$ oder $X_2$ Niederalkylthio, $X_2$ bzw. $X_1$ Wasserstoff und $Y_1$ und $Y_2$ zusammen eine zusätzliche Bindung bedeuten und $R_1$, $R_2$, $X_3$ und $X_4$ die unter der Formel I angegebene Bedeutung haben, umsetzt, oder

c) in einer Verbindung der Formel

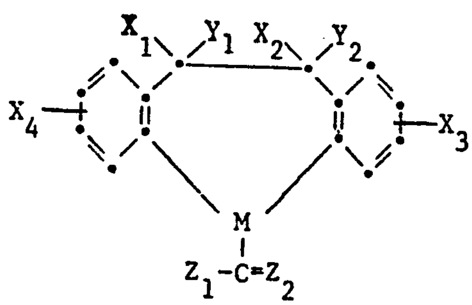

(V)

worin $Z_1$ eine verätherte Mercaptogruppe und $Z_2$ eine Gruppe der Formel n = $NR_1$, $[=NHR_1]^{\oplus}A^{\ominus}$ oder $[=N(R_1)(R_2)]^{\oplus}A^{\ominus}$, in der $A^{\ominus}$ ein Säureanion bedeutet, die $Z_1$—$C(=Z_2)$-Gruppe zu der entsprechenden $O=C(NR_1R_2)$-

Gruppe hydrolysiert und gewünschtenfalls eine Verbindung der allgemeinen Formel I, worin $X_1$ bzw. $X_2$ Niederalkoxy bedeutet und $Y_1$ und $Y_2$ gemeinsam eine zusätzliche Bindung darstellen, zur entsprechenden Verbindung, worin $X_1$ Hydroxy ist, bzw. zur tautomeren Oxoverbindung hydrolysiert und diese gewünschtenfalls zur entsprechenden Verbindung mit Hydroxy $X_1$ und Wasserstoff $Y_1$, $X_2$ und $Y_2$ reduziert oder eine Verbindung der allgemeinen Formel I, in welcher als $X_1$, $X_2$ und/oder $X_3$ Niederalkylthio bedeutet, zur entsprechenden Verbindung der allgemeinen Formel I mit Niederalkylsulfonyl oder Niederalkylsulfinyl als $X_1$, $X_2$ und/oder $X_3$ oxidiert.

21. Verfahren zur Herstellung neuer 5H-Dibenz[b,f]azepin-5-carboxamide der allgemeinen Formel I

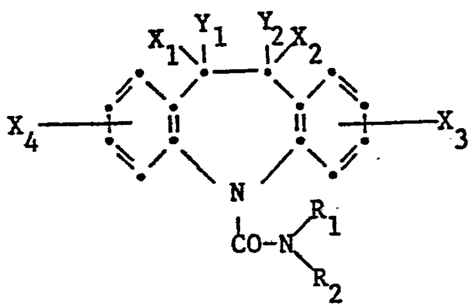

(I),

worin $R_1$ und $R_2$ unabhängig voneinander Wasserstoff oder Niederalkyl oder unter sich verbunden Niederalkylen oder Aethylenoxyäthylen, eines der beiden Symbole $X_1$ und $X_2$ und/oder das Symbol $X_3$ Niederalkylthio, Niederalkylsulfinyl, Nideralkylsulfonyl oder Trifluormethyl und $X_3$ auch Halogen bis Atomnummer 35, Diniederalkylsulfamoyl, Niederalkyl, Cyano oder Niederalkoxy bedeutet und das bzw. die beiden restlichen der Symbole $X_1$, $X_2$ und $X_3$ Wasserstoff bedeuten, jedoch $X_1$ oder $X_2$, sofern mindestens einer der Reste $R_1$ und $R_2$ von Wasserstoff und Niederalkyl oder einer der Reste $X_3$ und $X_4$ von Wasserstoff verschieden ist und $Y_1$ und $Y_2$ eine zusätzliche Bindung darstellen, auch Niederalkoxy und $X_2$ bzw. $X_1$ Wasserstoff bedeuten kann, $X_4$ Wasserstoff bedeutet oder zugleich mit Halogen oder Niederalkyl als $X_3$ ebenfalls einen dieser Reste bedeuten kann, $Y_1$ und $Y_2$ zusammen eine zusätzliche Bindung bedeuten oder zugleich mit Wasserstoff als $X_1$ und $X_2$ auch je Wasserstoff bedeuten können, oder falls mindestens einer der Reste $R_1$ und $R_2$ von

Wasserstoff verschieden ist und $X_3$ und $X_4$ Wasserstoff bedeuten, $X_1$ auch Halogen bis Atomnummer 35 oder Cyano, $X_2$ Wasserstoff und $Y_1$ und $Y_2$ zusammen eine zusätzliche Bindung bedeuten können, dadurch gekennzeichnet, dass man

a) eine Verbindung der allgemeinen Formel II

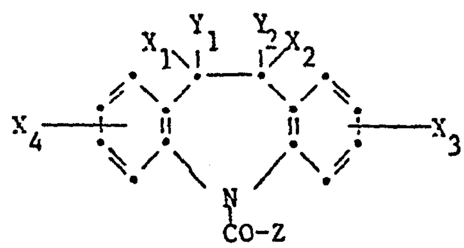

(II)

in welcher Z ein Halogenatom bedeutet, mit einer Verbindung der allgemeinen Formel III

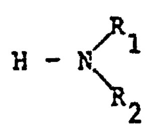

(III)

umsetzt, oder

b) eine Verbindung der allgemeinen Formel IV

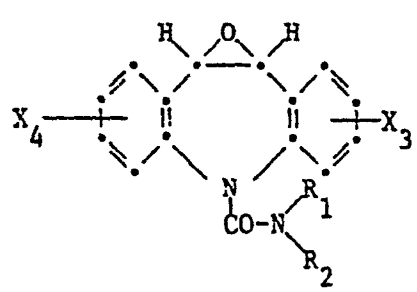

(IV)

in welcher $R_1$, $R_2$, $X_3$ und $X_4$ die unter der Formel I angegebene Bedeutung haben mit einem Niederalkylmercaptan oder einem Alkalimetall-niederalkylmercaptid zu einer Verbindung der allgemeinen Formel I umsetzt, in welcher $X_1$ oder $X_2$ Niederalkylthio, $X_2$ bzw. $X_1$ Wasserstoff und $Y_1$ und $Y_2$ zusammen eine zusätzliche Bindung bedeuten und $R_1$, $R_2$, $X_3$ und $X_4$ die unter der Formel I angegebene Bedeutung haben,

umsetzt und gewünschtenfalls eine Verbindu ıg der allgemeinen Formel I, in welcher als $X_1$, $X_2$ und/oder $X_3$ Niederalkylthio bedeutet, zur entsprechenden Verbindung der allgemeinen Formel I mit Niederalkylsulfonyl oder Niederalkylsulfinyl als $X_1$, $X_2$ und/oder $X_3$ oxidiert.

22. Verfahren gemäss Anspruch 21, dadurch gekennzeichnet, dass man Verbindungen der allgemeinen Formel I herstellt, worin $X_3$ con Cyano und $R_1$ und/oder $R_2$ von Wasserstoff bzw. mindestens einer der Reste $X_3$ und $X_4$ von Halogen verschieden ist, wenn $X_1$ und $X_2$ für Wasserstoff und $Y_1$ und $Y_2$ für eine zusätzliche Bindung stehen.

23. Verwendung von Verbindungen der allgemeinen Formel

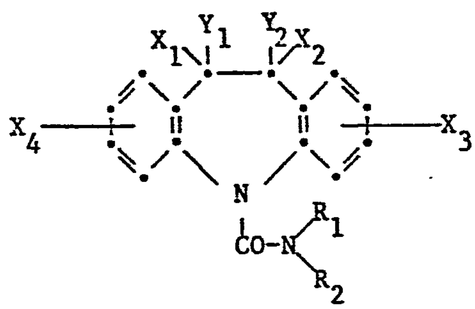

(I),

worin $R_1$ und $R_2$ unabhängig voneinander Wasserstoff oder Niederalkyl, oder unter sich verbunden Niederalkylen oder Aethylenoxyäthylen darstellen, eines der beiden Symbole $X_1$ und $X_2$ und/oder das Symbol $X_3$ Niederalkyl, Niederalkoxy, Niederalkylthio, Niederalkylsulfinyl, Niederalkylsulfonyl oder Trifluormethyl und $X_3$ auch Halogen bis Atomnummer 35, Cyano oder Diniederalkylsulfamoyl bedeutet und das bzw. die beiden restlichen der Symbole $X_1$, $X_2$ und $X_3$ Wasserstoff bedeuten, jedoch zugleich mit Niederalkyl als $X_1$ auch $X_2$ Niederalkyl bedeuten kann, oder, sofern mindestens einer der Reste $R_1$ und $R_2$ von Wasserstoff verschieden ist oder $Y_1$ und $Y_2$ Wasserstoff darstellen, $X_1$, $X_2$ und $X_3$ je Wasserstoff bedeuten können, $X_4$ Wasserstoff bedeutet oder zugleich mit Halogen oder Niederalkyl als $X_3$ ebenfalls einen dieser Reste bedeutet und $Y_1$ und $Y_2$ zusammen eine zusätzliche Bindung bedeuten oder, sofern $X_1$ und/oder $X_2$ für Niederalkyl oder

Wasserstoff stehen, beide Wasserstoff bedeuten, oder, falls jeweils mindestens einer der Reste $R_1$, $R_2$, $X_3$ und $X_4$ von Wasserstoff verschieden ist, $X_1$ bzw. $X_2$ auch Halogen bis Atomnummer 35 oder Cyano, $X_2$ bzw. $X_1$ Wasserstoff und $Y_1$ und $Y_2$ zusammen eine zusätzliche Bindung, oder $X_1$ und $Y_1$ zusammen den Oxorest und $X_2$ und $Y_2$ je Wasserstoff oder $X_1$ Hydroxy und $X_2$, $Y_1$ und $Y_2$ je Wasserstoff bedeuten können, oder worin $R_1$ Niederalkyl bedeutet, $R_2$, $X_3$, $X_4$, $Y_1$ und $Y_2$ Wasserstoff darstellen und $X_1$ und $X_2$ zusammen für Epoxy oder Epimethylen stehen, zur Prophylaxe und/oder Behandlung cerebraler Insuffizienz.

24. Verwendung von Verbindungen der Formel I gemäss Anspruch 23, worin $R_1$ und $R_2$ unabhängig voneinander Wasserstoff oder Niederalkyl, oder unter sich verbunden Niederalkylen oder Aethylenoxyäthylen darstellen, eines der beiden Symbole $X_1$ und $X_2$ und/oder das Symbol $X_3$ Niederalkyl, Niederalkoxy, Niederalkylthio, Niederalkylsulfinyl, Niederalkylsulfonyl oder Trifluormethyl und $X_3$ auch Halogen bis Atomnummer 35, Cyano oder Diniederalkylsulfamoyl bedeutet und das bzw. die beiden restlichen der Symbole $X_1$, $X_2$ und $X_3$ Wasserstoff bedeuten, jedoch zugleich mit Niederalkyl als $X_1$ auch $X_2$ Niederalkyl bedeuten kann, oder zugleich mit Wasserstoff als $Y_1$ und $Y_2$ auch $X_1$, $X_2$ und $X_3$ je Wasserstoff bedeuten können, $X_4$ Wasserstoff bedeutet oder zugleich mit Halogen oder Niederalkyl als $X_3$ ebenfalls einen dieser Reste bedeutet und $Y_1$ und $Y_2$ zusammen eine zusätzliche Bindung bedeuten, oder, sofern $X_1$ und/oder $X_2$ für Niederalkyl oder Wasserstoff stehen, beide Wasserstoff bedeuten, oder, falls jeweils mindestens einer der Reste $R_1$, $R_2$, $X_3$ und $X_4$ von Wasserstoff verschieden ist, $X_1$ bzw. $X_2$ auch Halogen bis Atomnummer 35 oder Cyano, $X_2$ bzw. $X_1$ Wasserstoff und $Y_1$ und $Y_2$ zusammen eine zusätzliche Bindung, oder $X_1$ und $Y_1$ zusammen den Oxorest und $X_2$ und $Y_2$ je Wasserstoff oder $X_1$ Hydroxy und $X_2$, $Y_1$ und $Y_2$ je Wasserstoff bedeuten können, zur Prophylaxe und/oder Behandlung cerebraler Insuffizienz.

25. Verwendung von
10,11-Dihydro-10-methyl-5H-dibenz[b,f]azepin-5-carboxamid,
10-Methyl-5H-dibenz[b,f]azepin-5-carboxamid,
10,11-Dimethyl-5H-dibenz[b,f]-azepin-5-carboxamid,
3,7-Dichlor- und 3,7-Dibrom-5H-dibenz[b,f]azepin-5-carboxamid,
N-Methyl-10,11-dihydro-10-oxo-5H-dibenz[b,f]azepin-5-carboxamid,
N,N-Dimethyl-10,11-dihydro-10-oxo-5H-dibenz[b,f]azepin-5-carboxamid,
N-Butyl-10,11-dihydro-10-oxo-5H-dibenz[b,f]azepin-5-carboxamid,
N,N-Dibutyl-10,11-dihydro-10-oxo-5H-dibenz[b,f]azepin-5-carboxamid,
N-Methyl-10,11-dihydro-10-hydroxy-5H-dibenz[b,f]azepin-5-carboxamid,
N,N-Dimethyl-10,11-dihydro-10-hydroxy-5H-dibenz[b,f]azepin-5-carboxamid,
N-Butyl-10,11-dihydro-10-hydroxy-5H-dibenz[b,f]azepin-5-carboxamid,
2-Cyano-5H-dibenz[b,f]azepin-5-carboxamid,
3-Cyano-5H-dibenz[b,f]azepin-5-carboxamid,
10,11-Dihydro-5H-dibenz[b,f]azepin-5-carboxamid,
N,N-Diäthyl-5H-dibenz[b,f]azepin-5-carboxamid,
5-[(1-Piperidinyl)-carbonyl]-5H-dibenz[b,f]azepin,
5-[(1-Pyrrolidinyl)-carbonyl]-5H-dibenz[b,f]azepin,
5-[(4-Morpholinyl)-carbonyl]-5H-dibenz[b,f]azepin,
10-Methoxy-5H-dibenz[b,f]azepin-5-carboxamid,
N-Methyl-10-methoxy-5H-dibenz[b,f]azepin-5-carboxamid,
N,N-Dimethyl-10-methoxy-5H-dibenz[b,f]azepin-5-carboxamid,
N-Butyl-10-methoxy-5H-dibenz[b,f]azepin-5-carboxamid,
N,N-Dibutyl-10-methoxy-5H-dibenz[b,f]azepin-5-carboxamid,
N-Methyl-1,1a,10b-tetrahydrocyclopropa[d]dibenz[b,f]azepin-6-carboxamid und/oder
N-Propyl-1a,10b-dihydro-6H-dibenz[b,f]oxiren[d]azepin-6-carboxamid,
zur Prophylaxe und/oder Behandlung cerebraler Insuffizienz.

26. Die nach dem Verfahren eines der Ansprüche 20 bis 22 erhältlichen neuen Verbindungen, verwendeten neuen Ausgangsstoffe und gebildete neue Zwischenprodukte.

Patentansprüche für den Vertragsstaat AT

1. Verfahren zur Herstellung neuer 5H-Dibenz[b,f]azepin-5-carboxamide der allgemeinen Formel I

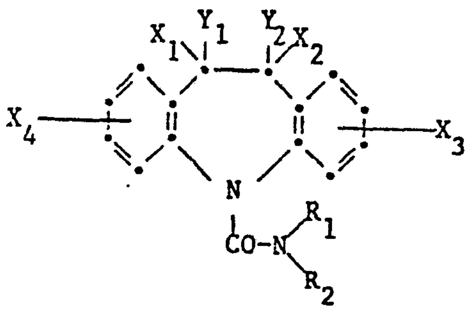

(I),

worin $R_1$ und $R_2$ unabhängig voneinander Wasserstoff oder Niederalkyl oder unter sich verbunden Niederalkylen oder Aethylenoxyäthylen, darstellen, eines der Symbole $X_1$ und $X_2$ Niederalkylthio, Niederalkylsulfinyl, Niederalkylsulfonyl, Trifluormethyl, Niederalkoxy, Halogen der Atomnummer bis und mit 35 oder Cyano und das andere Wasserstoff, Niederalkyl, Niederalkoxy, Niederalkylthio, Niederalkylsulfinyl, Niederalkylsulfonyl oder Trifluormethyl bedeutet oder beide Symbole $X_1$ und $X_2$ für Wasserstoff stehen, $X_3$ Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl darstellt oder, sofern mindestens einer der Reste $X_1$ und $X_2$ von Wasserstoff verschieden ist und zugleich mit Halogen oder Cyano als $X_1$ bzw. $X_2$ und Wasserstoff als $X_2$ bzw. $X_1$ $R_1$ Wasserstoff oder Niederalkyl und $R_2$ Niederalkyl oder $R_1$ gemeinsam mit $R_2$ Niederalkylen oder Aethylenoxyäthylen und zugleich mit Niederalkoxy als $X_1$ bzw. $X_2$ und Wasserstoff als $X_2$ bzw. $X_1$ $R_1$ gemeinsam mit $R_2$ Niederalkylen oder Aethylenoxyäthylen bedeutet, auch Wasserstoff bedeuten kann, $X_4$ Wasserstoff bedeutet oder zugleich mit Niederalkyl oder Halogen als $X_3$ ebenfalls einen dieser Reste darstellen kann, und die

Symbole $Y_1$ und $Y_2$, sofern eines der Symbole $X_1$ und $X_2$ Niederalkylthio, Niederalkylsulfinyl, Niederalkylsulfonyl oder Trifluormethyl und das andere Wasserstoff bedeutet oder beide Wasserstoff darstellen, jeweils für Wasserstoff stehen oder, sofern mindestens einer der Reste $R_1$, $R_2$, $X_1$ und $X_2$ von Wasserstoff oder $X_3$ von Cyano oder mindestens einer der Reste $X_3$ und $X_4$ von Halogen verschieden ist, gemeinsam eine zusätzliche Bindung darstellen, oder worin $R_1$ und $R_2$ gemeinsam für Niederalkylen oder Aethylenoxyäthylen stehen und $X_1$ gemeinsam mit $Y_1$ Oxo und $X_2$ sowie $Y_2$ Wasserstoff bzw. $X_1$ Hydroxy und $X_2$, $Y_1$ sowie $Y_2$ Wasserstoff darstellt oder $R_1$ und $R_2$ unabhängig voneinander Niederalkyl oder gemeinsam Niederalkylen oder Aethylenoxyäthylen bedeuten, $X_1$ oder Epimethylen und $Y_1$ sowie $Y_2$ jeweils Wasserstoff darstellt, $X_3$ für Wasserstoff, Niederalkyl, Niederalkoxy, Niederalkylthio, Niederalkylsulfinyl, Niederalkylsulfonyl, Trifluormethyl, Halogen der Atomnummer bis und mit 35, Cyano oder Diniederalkylsulfamoyl steht, und $X_4$ Wasserstoff bedeutet oder zugleich mit Niederalkyl oder Halogen als $X_3$ ebenfalls einen dieser Reste darstellt, dadurch gekennzeichnet, dass man

a) eine Verbindung der allgemeinen Formel II

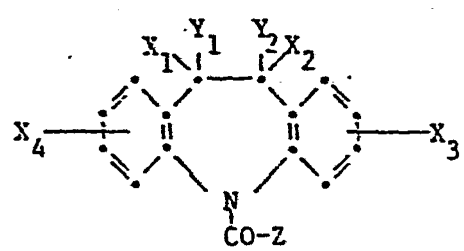

(II)

in welcher Z ein Halogenatom bedeutet, mit einer Verbindung der allgemeinen Formel III

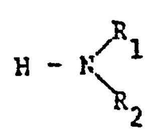

(III)

umsetzt, oder

b) eine Verbindung der allgemeinen Formel IV

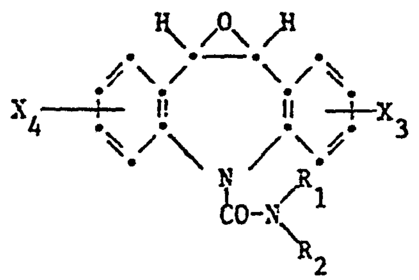

(IV)

in welcher $R_1$, $R_2$, $X_3$ und $X_4$ die unter der Formel I angegebene Bedeutung haben mit einem Niederalkylmercaptan oder einem Alkalimetall-niederalkylmercaptid zu einer Verbindung der allgemeinen Formel I umsetzt, in welcher $X_1$ oder $X_2$ Niederalkylthio, $X_2$ bzw. $X_1$ Wasserstoff und $Y_1$ und $Y_2$ zusammen eine zusätzliche Bindung bedeuten und $R_1$, $R_2$, $X_3$ und $X_4$ die unter der Formel I angegebene Bedeutung haben, umsetzt, oder

c) in einer Verbindung der Formel

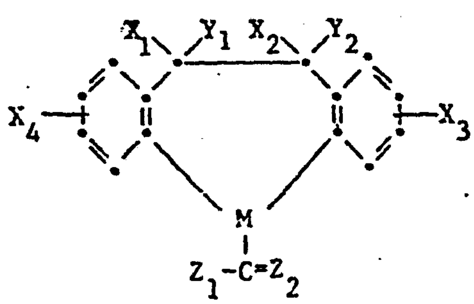

(V)

worin $Z_1$ eine verätherte Mercaptogruppe und $Z_2$ eine Gruppe der Formel n = $=NR_1$, $[=NHR_1]^{\oplus}A^{\ominus}$ oder $[=N(R_1)(R_2)]^{\oplus}A^{\ominus}$, in der $A^{\ominus}$ ein Säureanion bedeutet, die $Z_1—C(=Z_2)$-Gruppe zu der entsprechenden $O=C(NR_1R_2)$-

Gruppe hydrolysiert und gewünschtenfalls eine Verbindung der allgemeinen Formel I, worin $X_1$ bzw. $X_2$ Niederalkoxy bedeutet und $Y_1$ und $Y_2$ gemeinsam eine zusätzliche Bindung darstellen, zur entsprechenden Verbindung, worin $X_1$ Hydroxy ist, bzw. zur tautomeren Oxoverbindung hydrolysiert und diese gewünschtenfalls zur entsprechenden Verbindung mit Hydroxy $X_1$ und Wasserstoff $Y_1$, $X_2$ und $Y_2$ reduziert oder eine Verbindung der allgemeinen Formel I, in welcher als $X_1$, $X_2$ und/oder $X_3$ Niederalkylthio bedeutet, zur entsprechenden Verbindung der allgemeinen Formel I mit Niederalkylsulfonyl oder Niederalkylsulfinyl als $X_1$, $X_2$ und/oder $X_3$ oxidiert.

2. Verfahren gemäss Anspruch 1 zur Herstellung neuer 5H-Dibenz[b,f]-azepin-5-carboxamide der allgemeinen Formel I

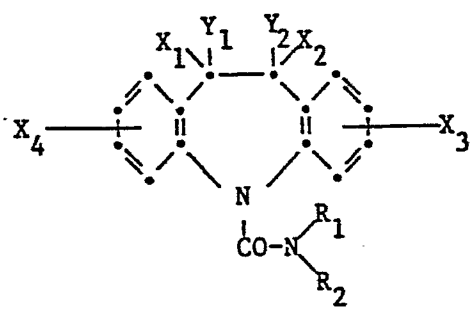

(I),

worin $R_1$ und $R_2$ unabhängig voneinander Wasserstoff oder Niederalkyl oder unter sich verbunden Niederalkylen oder Aethylenoxyäthylen, eines der beiden Symbole $X_1$ und $X_2$ und/oder das Symbol $X_3$ Niederalkylthio, Niederalkylsulfinyl, Nideralkylsulfonyl oder Trifluormethyl und $X_3$ auch Halogen bis Atomnummer 35, Diniederalkylsulfamoyl, Niederalkyl, Cyano oder Niederalkoxy bedeutet und das bzw. die beiden restlichen der Symbole $X_1$, $X_2$ und $X_3$ Wasserstoff bedeuten, jedoch $X_1$ oder $X_2$, sofern mindestens einer der Reste $R_1$ und $R_2$ von Wasserstoff und Niederalkyl oder einer der Reste $X_3$ und $X_4$ von Wasserstoff verschieden ist und $Y_1$ und $Y_2$ eine zusätzliche Bindung darstellen, auch Niederalkoxy und $X_2$ bzw. $X_1$ Wasserstoff bedeuten kann, $X_4$ Wasserstoff bedeutet oder zugleich mit Halogen oder Niederalkyl als $X_3$ ebenfalls einen dieser Reste bedeuten kann, $Y_1$ und $Y_2$ zusammen eine zusätzliche Bindung bedeuten oder zugleich mit Wasserstoff als $X_1$ und $X_2$ auch je Wasserstoff bedeuten können, oder falls mindestens einer der Reste $R_1$ und $R_2$ von

Wasserstoff verschieden ist und $X_3$ und $X_4$ Wasserstoff bedeuten, $X_1$ auch Halogen bis Atomnummer 35 oder Cyano, $X_2$ Wasserstoff und $Y_1$ und $Y_2$ zusammen eine zusätzliche Bindung bedeuten können, dadurch gekennzeichnet, dass man

a) eine Verbindung der allgemeinen Formel II

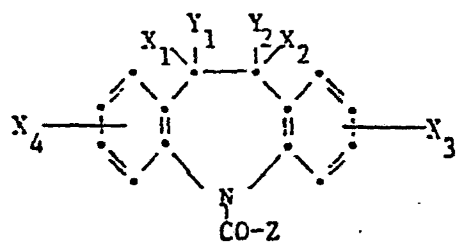

(II)

in welcher Z ein Halogenatom bedeutet, mit einer Verbindung der allgemeinen Formel III

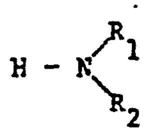

(III)

umsetzt, oder

b) eine Verbindung der allgemeinen Formel IV

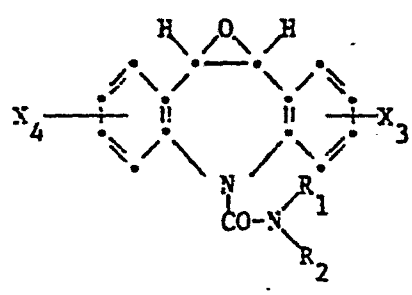

(IV)

in welcher $R_1$, $R_2$, $X_3$ und $X_4$ die unter der Formel I angegebene Bedeutung haben mit einem Niederalkylmercaptan oder einem Alkalimetall-niederalkylmercaptid zu einer Verbindung der allgemeinen Formel I umsetzt, in welcher $X_1$ oder $X_2$ Niederalkylthio, $X_2$ bzw. $X_1$ Wasserstoff und $Y_1$ und $Y_2$ zusammen eine zusätzliche Bindung bedeuten und $R_1$, $R_2$, $X_3$ und $X_4$ die unter der Formel I angegebene Bedeutung haben,

umsetzt und gewünschtenfalls eine Verbindung der allgemeinen Formel I, in welcher als $X_1$, $X_2$ und/oder $X_3$ Niederalkylthio bedeutet, zur entsprechenden Verbindung der allgemeinen Formel I mit Niederalkylsulfonyl oder Niederalkylsulfinyl als $X_1$, $X_2$ und/oder $X_3$ oxidiert.

3. Verfahren gemäss Anspruch 2, dadurch gekennzeichnet, dass man Verbindungen der allgemeinen Formel I herstellt, worin $X_3$ con Cyano und $R_1$ und/oder $R_2$ von Wasserstoff bzw. mindestens einer der Reste $X_3$ und $X_4$ von Halogen verschieden ist, wenn $X_1$ und $X_2$ für Wasserstoff und $Y_1$ und $Y_2$ für eine zusätzliche Bindung stehen.

4. Verfahren gemäss Anspruch 1, dadurch gekennzeichnet, dass man Verbindungen der allgemeinen Formel I, worin $R_1$ und $R_2$ unabhängig voneinander Wasserstoff oder Niederalkyl oder unter sich verbunden Niederalkylen oder Aethylenoxyäthylen bedeuten, eines der beiden Symbole $X_1$ und $X_2$ Niederalkylthio, Niederalkylsulfinyl, Niederalkylsulfonyl oder Trifluormethyl und das andere Wasserstoff darstellt, $X_3$ Wasserstoff, Niederalkylthio, Niederalkylsulfinyl, Niederalkylsulfonyl, Trifluormethyl, Cyano, Halogen bis Atomnummer 35, Diniederalkylsulfamoyl, Niederalkyl oder Niederalkoxy bedeutet, $X_4$ Wasserstoff bedeutet oder zugleich mit Halogen oder Niederalkyl als $X_3$ ebenfalls einen dieser Reste bedeuten kann und $Y_1$ und $Y_2$ zusammen eine zusätzliche Bindung oder jeweils Wasserstoff darstellen, herstellt.

5. Verfahren gemäss einem der Ansprüche 1 bis 3, dadurch gekennzeichnet, dass man Verbindungen der allgemeinen Formel I, worin $R_1$ und $R_2$ unabhängig voneinander Wasserstoff oder Niederalkyl oder unter sich verbunden Niederalkylen oder Aethylenoxyäthylen, $X_1$ und $X_2$ Wasserstoff bedeuten, $X_3$ Halogen der Atomnummer bis und mit 35, Niederalkyl, Niederalkoxy, Niederalkylthio, Niederalkylsulfinyl, Niederalkylsulfonyl, Trifluormethyl, Cyano oder Diniederalkylsulfamoyl bedeutet, $X_4$ Wasserstoff bedeutet oder zugleich kann und $Y_1$ und $Y_2$ jeweils wasserstoff oder, sofern mindestens einer der Reste $R_1$ und $R_2$ von Wasserstoff oder $X_3$ von Cyano oder $X_4$ von Halogen verschieden

ist, zusammen eine zusätzliche Bindung darstellen, herstellt.

6. Verfahren gemäss einem der Ansprüche 1 bis 3, dadurch gekennzeichnet, dass man Verbindungen der allgemeinen Formel I, worin $R_1$ und $R_2$ unabhängig voneinander Wasserstoff oder Niederalkyl oder unter sich verbunden Niederalkylen oder Aethylenoxyäthylen darstellen, $X_1$ und $Y_1$ gemeinsam Oxo und $X_2$ und $Y_2$ jeweils Wasserstoff bzw. $X_1$ Niederalkoxy oder Hydroxy und $X_2$, $Y_1$ und $Y_2$ jeweils Wasserstoff darstellen, $X_3$ für Halogen der Atomnummer bis und mit 35, Niederalkyl, Niederalkoxy, ferner Niederalkylthio, Niederalkylsulfinyl, Niederalklysulfonyl, Trifluormethyl, Cyano oder Diniederalkylsulfamoyl oder, sofern $R_1$ und $R_2$ gemeinsam Niederalkylen oder Aethylenoxyäthylen darstellen, für Wasserstoff steht und $X_4$ Wasserstoff bedeutet oder zugleich mit Halogen oder Niederalkyl als $X_3$ ebenfalls einen dieser Reste bedeuten kann, herstellt.

7. Verfahren gemäss Anspruch 1, dadurch gekennzeichnet, dass man Verbindungen der allgemeinen Formel I, worin $R_1$, $R_2$, $X_1$ und $X_2$ Wasserstoff bedeuten, $X_3$ Niederalkylthio, Niederalkylsulfinyl oder Niederalkylsulfonyl mit jeweils bis und mit 4 C-Atomen, Diniederlakylsulfamoyl mit bis und mit 4 C-Atomen in jedem Alkylteil, Niederalkyl oder Niederalkoxy mit bis und mit 4 C-Atomen, Halogen der Atomnummer bis und mit 35, oder Trifluormethyl bedeutet, $X_4$ für Wasserstoff steht oder zugleich mit Niederalkyl oder Halogen als $X_3$ auch für Niederalkyl stehen kann und $Y_1$ und $Y_2$ jeweils Wasserstoff oder zusammen eine zusätzliche Bindung darstellen, herstellt.

8. Verfahren gemäss Anspruch 1, dadurch gekennzeichnet, dass man Verbindungen der allgemeinen Formel I, worin $R_1$ und $R_2$ unabhängig voneinander Wasserstoff oder Niederalkyl darstellen, welches je bis und mit 4 C-Atome aufweist, einer der Reste $X_1$ und $X_2$ Niederalkylthio, Niederalkylsulfinyl oder Niederalkylsulfonyl mit jeweils bis und

mit 4 C-Atomen oder Trifluormethyl oder, sofern mindestens einer der Reste $R_1$ und $R_2$ Niederalkyl darstellt, Halogen der Atomnummer bis und 35, oder Cyano bedeutet und der andere sowie $X_3$ und $X_4$ Wasserstoff ist und $Y_1$ und $Y_2$ zusammen eine zusätzliche Bindung darstellen, herstellt.

9. Verfahren gemäss Anspruch 1, dadurch gekennzeichnet, dass man Verbindungen der allgemeinen Formel I, worin 5 der Symbole $R_1$, $R_2$, $X_1$, $X_2$, $X_3$ und $X_4$ Wasserstoff darstellen und ein davon verschiedener Rest $R_1$ und/oder $R_2$ Niederalkyl mit bis und mit 4 C-Atomen, ein davon verschiedener Rest $X_1$ oder $X_2$ Niederalkylthio, Niederalkylsulfinyl odr Niederalkylsulfonyl mit jeweils bis und mit 4 C-Atomen oder Trifluormethyl bzw. ein davon verschiedener Rest $X_3$ Niederalkylthio, Niederalkylsulfinyl oder Niederalkylsulfonyl mit jeweils bis und mit 4 C-Atomen, Diniederalkylsulfamoyl mit bis und mit 4 C-Atomen in jedem Alkylteil, Niederalkyl oder Niederalkoxy mit bis und mit 4 C-Atomen, Halogen der Atomnummer bis und mit 35 oder Trifluormethyl bedeutet und $X_1$ und $Y_2$ zusammen eine zusätzliche Bindung darstellen, herstellt.

10. Verfahren gemäss einem der Ansprüche 1 bis 3, dadurch gekennzeichnet, dass man 3-Methylsulfonyl-5H-dibenz[b,f]azepin-5-carboxamid,
3-Methylthio-5H-dibenz[b,f]azepin-5-carboxamid,
3-Methoxy-5H-dibenz[b,f]azepin-5-carboxamid,
3-(Dimethylsulfamoyl)-5H-dibenz[b,f]azepin-5-carboxamid,
3-Cyano-5H-dibenz[b,f]azepin-5-carboxamid,
2-Cyano-5H-dibenz[b,f]azepin-5-carboxamid,
N-Methyl-5H-dibenz[b,f]azepin-5-carboxamid,
N-10-Dimethyl-5H-dibenz[b,f]azepin-5-carboxamid,
10-Methylthio-5H-dibenz[b,f]azepin-5-carboxamid oder
10-Methylsulfonyl-5H-dibenz[b,f]azepin-5-carboxamid
herstellt.

11. Verfahren nach Anspruch 1, dadurch gekennzeichnet, dass man
10-Trifluormethyl-5H-dibenz[b,f]azepin-5-carboxamid,
10-Trifluormethyl-10,11-dihydro-5H-dibenz[b,f]azepin-5-carboxamid,
10-Methoxy-11-methyl-5H-dibenz[b,f]azepin-5-carboxamid,
10-Cyano-5H-dibenz[b,f]azepin-5-(N-methyl)-carboxamid,
3,7-Dichlor-10,11-dihydro-5H-dibenz[b,f]azepin-5-carboxamid,
3,7-Dimethyl-10,11-dihydro-5H-dibenz[b,f]azepin-5-carboxamid,
2,8-Dimethyl-10,11-dihydro-5H-dibenz[b,f]azepin-5-carboxamid,
3-Methylthio-10,11-dihydro-5H-dibenz[b,f]azepin-5-carboxamid,
3-Dimethylsulfamoyl-10,11-dihydro-5H-dibenz[b,f]azepin-5-carboxamid,
10-Methylsulfinyl-5H-dibenz[b,f]azepin-5-carboxamid,
3-Chlor-5H-dibenz[b,f]azepin-5-carboxamid,
5-[(1-Piperidinyl)-carbonyl]-10,11-dihydro-10-oxo-5H-dibenz[b,f]azepin,
5-[(1-Piperidinyl)-carbonyl]-10,11-dihydro-10-hydroxy-5H-dibenz[b,f]-azepin,
3-Methylsulfonyl-10,11-dihydro-5H-dibenz[b,f]azepin-5-carboxamid oder
10-Cyano-11-methoxy-5H-dibenz[b,f]azepin-5-carboxamid herstellt.

12. Verfahren zur Herstellung pharmazeutischer Präparate, dadurch gekennzeichnet, dass man eine gemäss einem der Ansprüche 2, 3, 5 bis 7 und 10 erhältliche Verbindung mit üblichen pharmazeutischen Hilfs- und/oder Trägerstoffen vermischt.

13. Verfahren zur Herstellung pharmazeutischer Präparate, dadurch gekennzeichnet, dass man eine gemäss einem der Ansprüche 1, 3, 4, 7 bis 9 und 11 erhältliche Verbindung mit üblichen pharmazeutischen Hilfs- und/oder Trägerstoffen vermischt.

14. Verfahren zur Herstellung pharmazeutischer Präparate, dadurch gekennzeichnet, dass man eine Verbindung der allgemeinen Formel I,

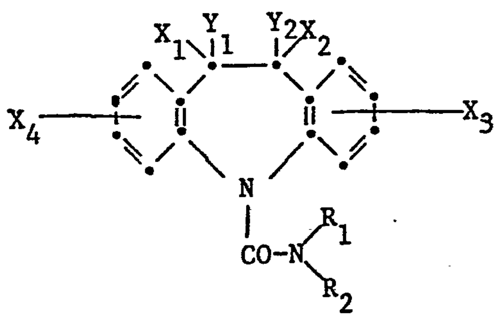

(I),

worin $X_1$ Niederalkoxy bedeutet, $R_1$ und $R_2$ Wasserstoff oder Niederalkyl, $X_2$, $X_3$ und $X_4$ je Wasserstoff und $Y_1$ und $Y_2$ zusammen eine zusätzliche Bindung bedeuten, mit üblichen pharmazeutischen Hilfs- und/oder Trägerstoffen vermischt.

15. Verfahren gemäss Anspruch 14 zur Herstellung pharmazeutischer Präparate, dadurch gekennzeichnet, dass man

a) eine Verbindung der allgemeinen Formel II

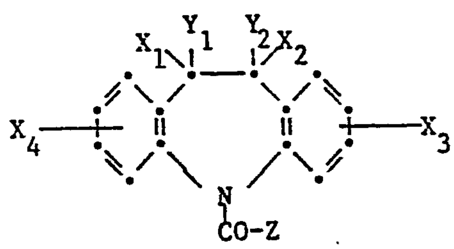

(II)

in welcher Z ein Halogenatom bedeutet, mit einer Verbindung der allgemeinen Formel III

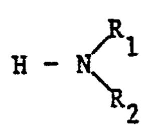

(III)

umsetzt, oder

c) in einer Verbindung der Formel

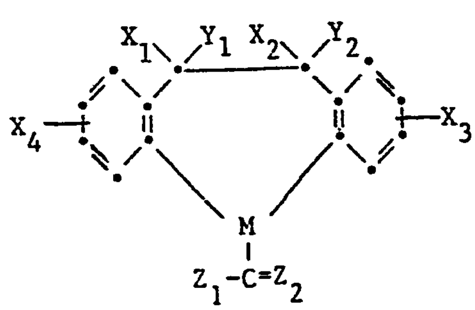

(V) ,

wobei $X_1$ Niederalkoxy bedeutet, $R_1$ und $R_2$ Wasserstoff oder Niederalkyl $X_2$, $X_3$ und $X_4$ je Wasserstoff und $Y_1$ und $Y_2$ zusammen eine zusätzliche Bindung bedeuten, $Z_1$ eine verätherte Mercaptogruppe und Z eine Gruppe der Formel n = $NR_1$, $[=NHR_1]^{\oplus}A^{\ominus}$ oder $[=N(R_1)(R_2)]^{\oplus}A^{\ominus}$, in der $A^{\ominus}$ ein Säureanion bedeutet, die $Z_1$-C(=$Z_2$)-Gruppe zu der entsprechenden O=C ($NR_1R_2$)-Gruppe hydrolysiert und das Reaktionsprodukt mit üblichen pharmazeutischen Hilfs- und/oder Trägerstoffen vermischt.

16. Verfahren gemäss Anspruch 14 oder 15 zur Herstellung pharmazeutischer Präparate enthaltend 10-Methoxy-5H-dibenz[b,f]azepin-5-carboxamid, N-Methyl-10-methoxy-5H-dibenz[b,f]azepin-5-carboxamid, N,N-Dimethyl-10-methoxy-5H-dibenz[b,f]-azepin-5-carboxamid, N-Butyl-10-methoxy-5H-dibenz[b,f]-azepin-5-carboxamid, oder N,N-Dibutyl-10-methoxy-5H-dibenz[b,f]azepin-5-carboxamid.

0108715

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 83810468.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A,D | CH - A - 403 767 (GEIGY)<br>* Patentanspruch; Seite 1, Zeilen 40-43 *<br>-- | 1,14, 20 | C 07 D 223/26<br>C 07 D 223/28<br>C 07 D 223/22<br>A 61 K 31/55 |
| A | DE - B2 - 2 011 045 (CIBA-GEIGY)<br>* Spalte 1, Zeile 48 - Spalte 3, Zeile 30 *<br>-- | 1,14, 18,20 | |
| A | DE - B2 - 2 011 087 (CIBA-GEIGY)<br>* Spalte 1, Zeile 47 - Spalte 3, Zeile 15 *<br>-- | 1,14, 18,20 | |
| A,D | GB - A - 906 452 (GEIGY)<br>* Seite 1, Zeilen 9-59 *<br>-- | 1,14, 20 | RECHERCHIERTE SACHGEBIETE (Int. Cl. 3) |
| A | EP - A1 - 0 011 603 (CIBA-GEIGY)<br>* Patentansprüche *<br>-- | 1,14, 18,20 | C 07 D 223/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-22,26

Unvollständig recherchierte Patentansprüche: -

Nicht recherchierte Patentansprüche: 23-25

Grund für die Beschränkung der Recherche:

Verfahren zur therapeutischen Behandlung des menschlichen oder tierischen Körpers, Art. 52(4)EPÜ

| Recherchenort | Abschlußdatum der Recherche | Prüfer |
|---|---|---|
| WIEN | 30-12-1983 | LUX |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82

0108715

Europäisches Patentamt

# EUROPÄISCHER TEILRECHERCHENBERICHT

Nummer der Anmeldung

EP 83810468.5

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| A | DE - A1 - 2 542 335 (CIBA-GEIGY)<br>* Seite 1, Zeile 6 - Seite 3, Zeile 9 *<br>-- | 1,14, 20 | |
| A | GB - A - 1 540 588 (DSO PHARMACHIM)<br>* Patentanspruch 1 *<br>---- | 1,20 | |
| | | | RECHERCHIERTE SACHGEBIETE (Int. Cl.3) |